(19) Europäisches Patentamt
European Patent Office
Office européen des brevets

(11) Publication number: 0 253 179 B1

(12) **EUROPEAN PATENT SPECIFICATION**

(45) Date of publication of patent specification: **04.12.91**

(51) Int. Cl.5: **C07F 9/547**, C07F 9/40, A61K 31/66

(21) Application number: **87109254.0**

(22) Date of filing: **26.06.87**

(54) **A-acylamino aminoalkyl phophonate angiotensin converting enzyme inhibitors.**

(30) Priority: **14.07.86 US 885560**

(43) Date of publication of application: **20.01.88 Bulletin 88/03**

(45) Publication of the grant of the patent: **04.12.91 Bulletin 91/49**

(84) Designated Contracting States: **BE CH DE FR GB IT LI NL SE**

(56) References cited:
**EP-A- 0 083 172**
**EP-A- 0 097 534**

(73) Proprietor: **E.R. Squibb & Sons, Inc.**
**Lawrenceville-Princeton Road**
**Princeton, N.J. 08540-4000(US)**

(72) Inventor: **Karanewsky, Donald Steven**
**8 Charred Oak Lane**
**East Windsor New Jersey(US)**
Inventor: **Petrillo, Edward William, Jr.**
**5 Walking Purchase Drive**
**Pennington New Jersey(US)**

(74) Representative: **Perani, Aurelio et al**
**c/o JACOBACCI-CASETTA & PERANI S.N.C.**
**Via Visconti di Modrone 7**
**I-20122 Milano(IT)**

**EP 0 253 179 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Karanewsky et al. in United States Patent 4,452,790 disclose angiotensin converting enzyme inhibitors of the formula

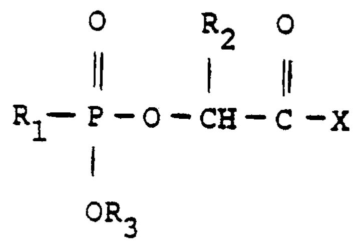

wherein $R_1$ is alkyl, substituted alkylene, or

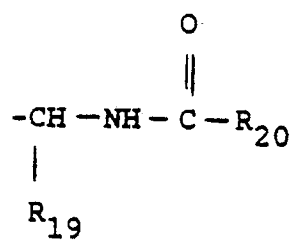

$R_{19}$ is defined as hydrogen, lower alkyl, cycloalkyl, aryl, aralkyl, heterocyclo, and heterocycloalkylene.

Karanewsky et al. in United States Patent 4,555,506 disclose angiotensin converting enzyme inhibitors of the formula

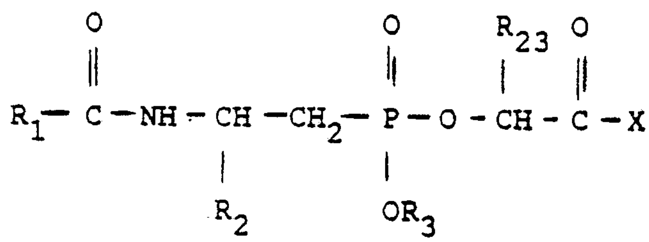

wherein $R_1$ and $R_2$ are independently selected from hydrogen, lower alkyl, aryl, heterocyclo, cycloalkyl, or alkyl substituted with an amino, halo, cycloalkyl, aryl, or heterocyclo.

This invention is directed to new α-acylamino aminoalkyl phosphonate substituted amino or imino acids of formula I and salts thereof

(I)

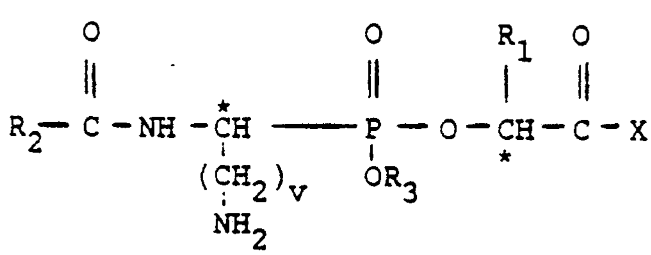

$R_1$ is hydrogen, lower alkyl, $-(CH_2)_r-Cl$,

$-(CH_2)_r-Br$, $-(CH_2)_r-F$, $CF_3$, 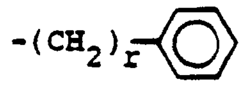

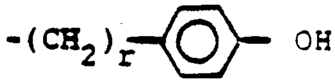 , 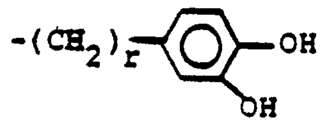 ,

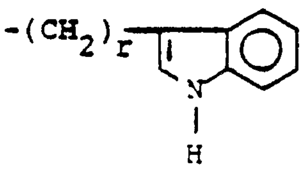 , 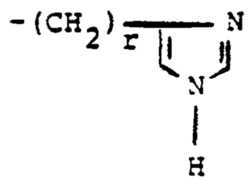 ,

$-(CH_2)_r$-cycloalkyl, $-(CH_2)_r-NH_2$, $-(CH_2)_r-SH$,

$-(CH_2)_r$-S-lower alkyl, 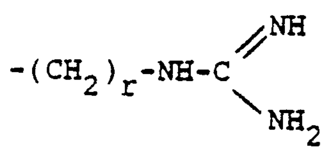 , or

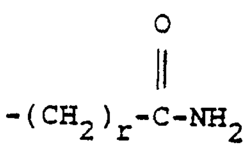 .

$R_2$ is straight or branched chain alkyl of 1 to 10 carbons, $-(CH_2)_q$-cycloalkyl,

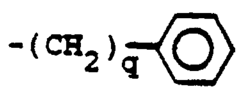 ,

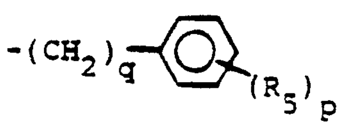 , 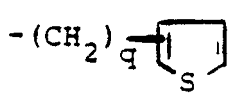 ,

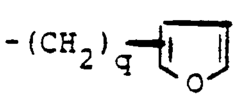 or 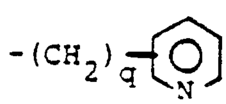 .

$R_3$ is hydrogen, lower alkyl, benzyl, alkali metal salt ion, alkaline earth metal salt ion, or

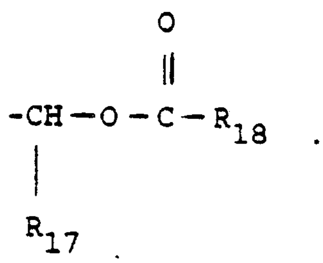

v is an integer from 3 to 5.
R is an integer from 1 to 7.
Q is zero or an integer from 1 to 7.
X is an amino or imino acid or ester of the formula

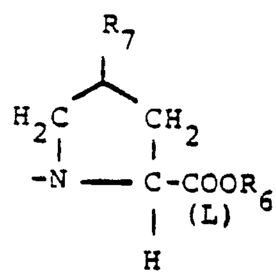
,
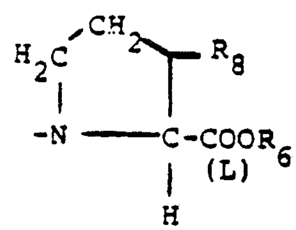
,

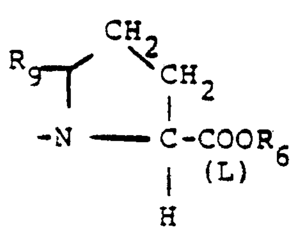
,
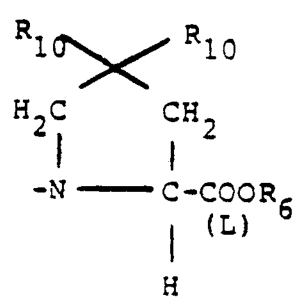
,

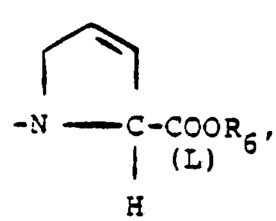

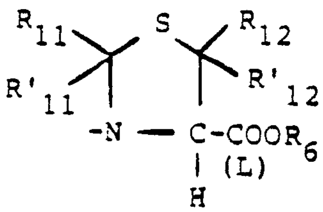 , 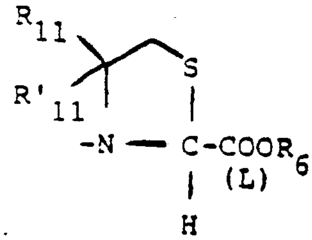 ,

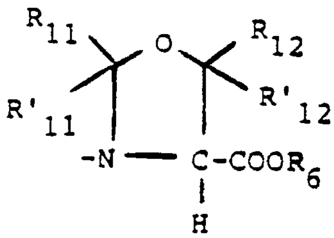

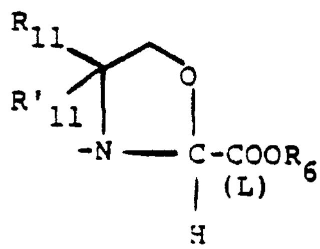

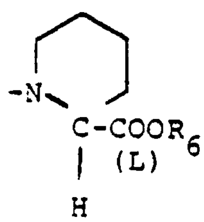 , 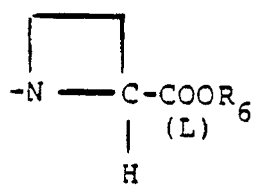 ,

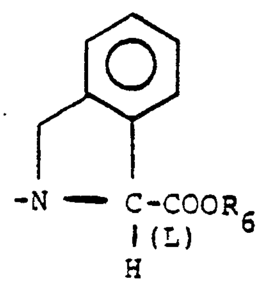 , 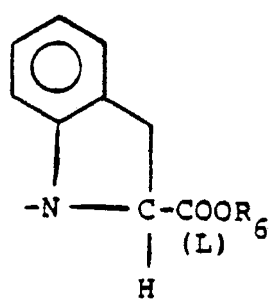 ,

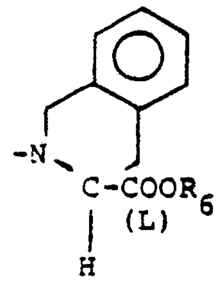
,
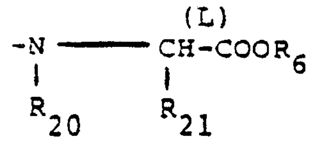

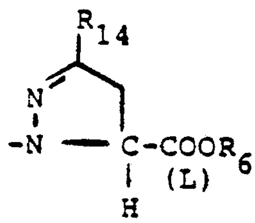
,
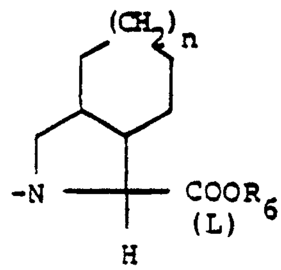
,

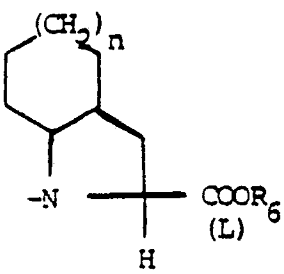
,
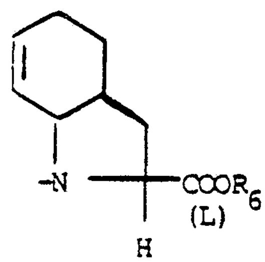
,

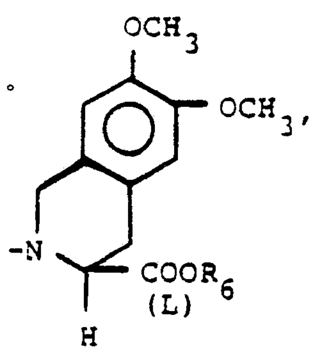

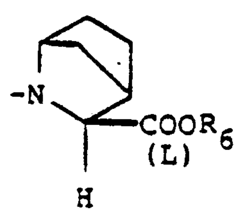
,

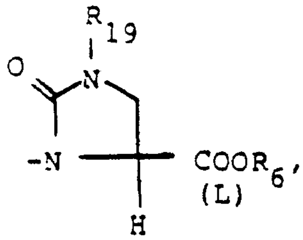 , or 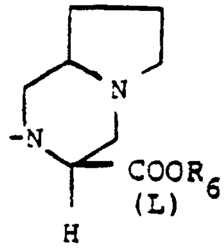 .

$R_7$ is hydrogen, lower alkyl, halogen, hydroxy,

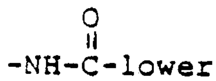 , amino, 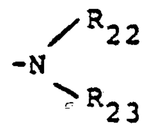 ,

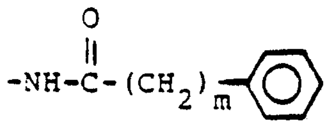 , 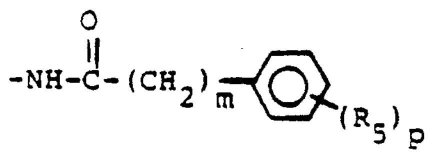 ,

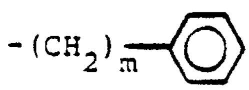 , 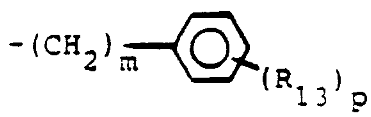 ,

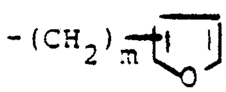 , 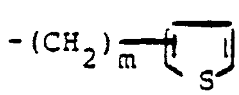 , 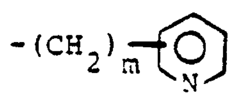 ,

a 1- or 2-naphthyl of the formula

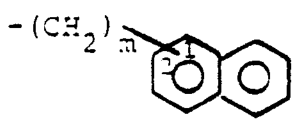 ,

a substituted 1- or 2-naphthyl of the formula

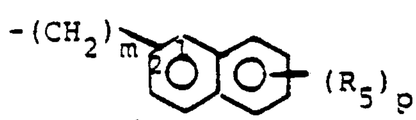 ,

$-(CH_2)_m$-cycloalkyl, 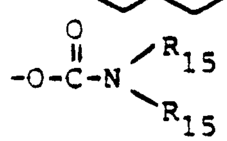 , -O-lower alkyl,

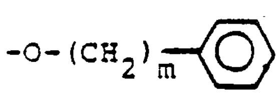 , 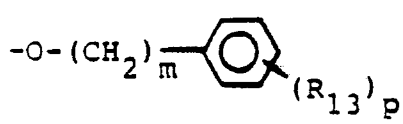 ,

a 1- or 2- naphthyloxy of the formula

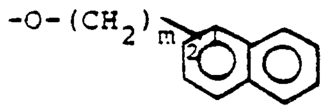 ,

a substituted 1- or 2-naphthyloxy of the formula

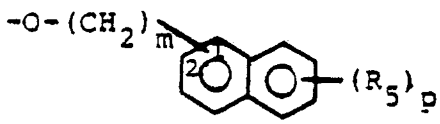 ,

-S-lower alkyl,

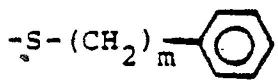 ,

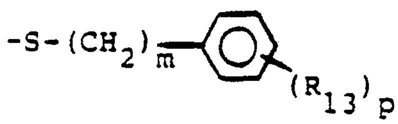 ,

a 1- or 2-naphthylthio of the formula

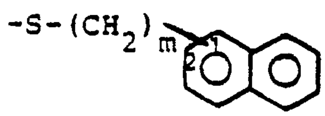 ,

or a substituted 1- or 2-naphthylthio of the formula

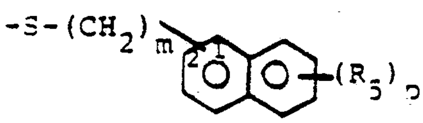 .

$R_8$ is lower alkyl, halogen,

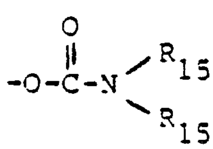 ,

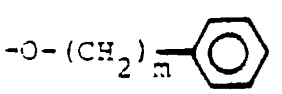 , 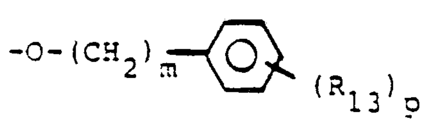 ,

-O-lower alkyl, a 1- or 2-naphthyloxy of the formula

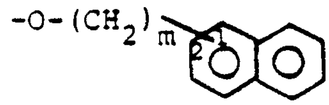,

a substituted 1- or 2-naphthyloxy of the formula

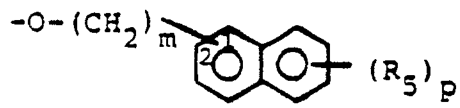,

-S-lower alkyl,

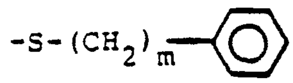,

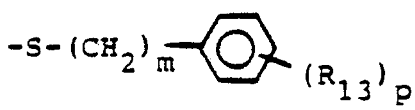,

a 1- or 2-naphthylthio of the formula

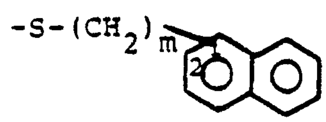,

or a substituted 1- or 2-naphthylthio of the formula

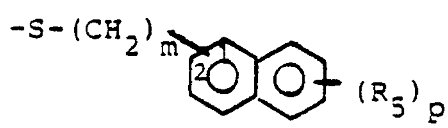.

$R_9$ is lower alkyl, keto,

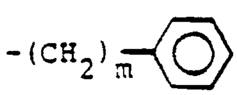,

or

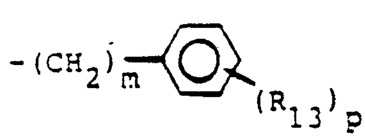.

$R_{10}$ is halogen or Y-$R_{16}$.

$R_{11}$, $R'_{11}$, $R_{12}$ and $R'_{12}$ are independently selected from hydrogen and lower alkyl or $R'_{11}$, $R_{12}$ and $R'_{12}$ are hydrogen and $R_{11}$ is

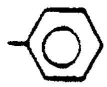 or 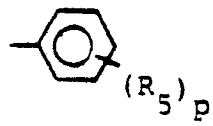 .

$R_{13}$ is lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl.

$R_5$ is lower alkyl of 1 to 4 carbons, lower alkoxy of 1 to 4 carbons, lower alkylthio of 1 to 4 carbons, chloro, bromo, fluoro, trifluoromethyl or hydroxy.

m is zero, one, two, three, or four.

p is one, two or three provided that p is more than one only if $R_{13}$ or $R_5$ is methyl, methoxy, chloro, bromo, or fluoro.

$R_{14}$ is hydrogen, lower alkyl,

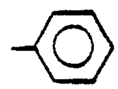

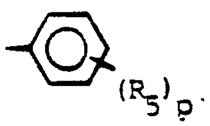 , 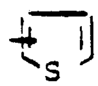 , 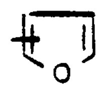 , or 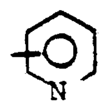 .

$R_{15}$ is hydrogen or lower alkyl of 1 to 4 carbons.

Y is oxygen or sulfur.

$R_{16}$ is lower alkyl of 1 to 4 carbons,

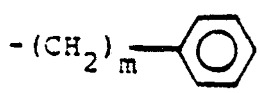 , 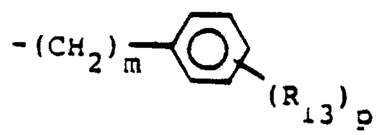 ,

or the $R_{16}$ groups join to complete an unsubstituted 5- or 6-membered ring or said ring in which one or more of the carbons has a lower alkyl of 1 to 4 carbons or a di(lower alkyl of 1 to 4 carbons) substituent.

$R_{17}$ is hydrogen, lower alkyl, cycloalkyl, or phenyl.

$R_{18}$ is hydrogen, lower alkyl, lower alkoxy, or phenyl.

n is zero, one, or two.

$R_{19}$ is lower alkyl or

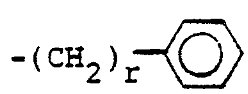 .

$R_{20}$ is hydrogen, lower alkyl,

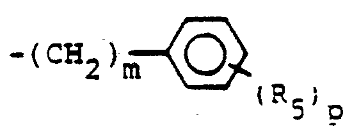 ,

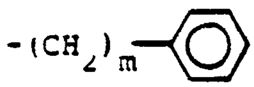 , 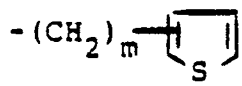 , 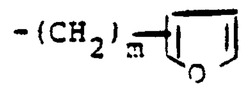 ,

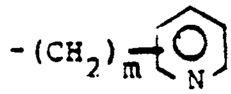 , 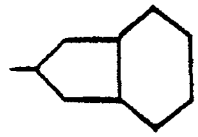 , 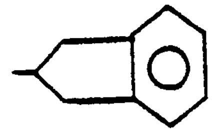 , or

$-(CH_2)_m$-cycloalkyl.

$R_{21}$ is hydrogen, lower alkyl,

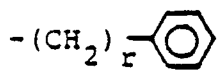 ,

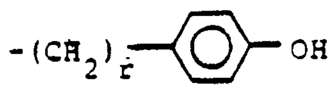 , $-(CH_2)_r-OH$, 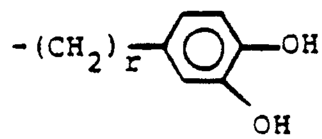 ,

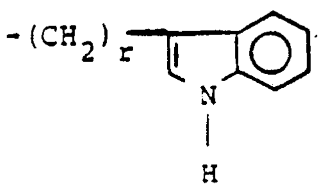 , 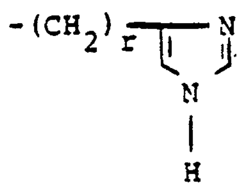 ,

$-(CH_2)_r-NH_2$ , $-(CH_2)_r-SH$ , $-(CH_2)_r$-S-lower alkyl,

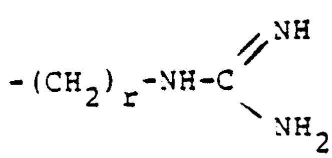 , or 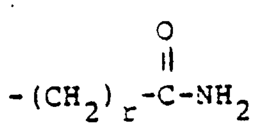 .

$R_{22}$ is lower alkyl, benzyl, or phenethyl.
$R_{23}$ is hydrogen, lower alkyl, benzyl, or phenethyl.
$R_6$ is hydrogen, lower alkyl, benzyl, benzhydryl, alkali metal salt ion, alkaline earth metal salt ion,

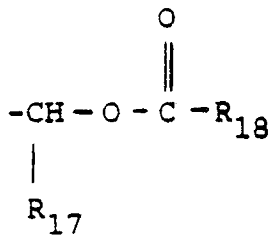

, or $-(CH_2)_2Si(CH_3)_3$ .

This invention in its broadest aspects relates to the amino and imino acid and ester compounds of formula I and to compositions and the method of using such compounds as pharmaceutical agents.

The term lower alkyl used in defining various symbols refers to straight or branched chain radicals having up to seven carbons. The preferred lower alkyl groups are up to four carbons with methyl and ethyl most preferred. Similarly the terms lower alkoxy and lower alkylthio refer to such lower alkyl groups attached to an oxygen or sulfur.

The term cycloalkyl refers to saturated rings of 4 to 7 carbon atoms with cyclopentyl and cyclohexyl being most preferred.

The term halogen refers to chloro, bromo and fluoro.

The symbols

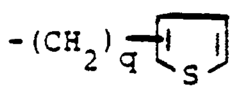 , 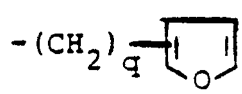 , and 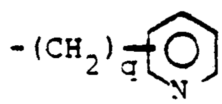

represent that the alkylene bridge is attached to an available carbon atom.

The compounds of formula I can be prepared by coupling a phosphonic acid of the formula

(II)

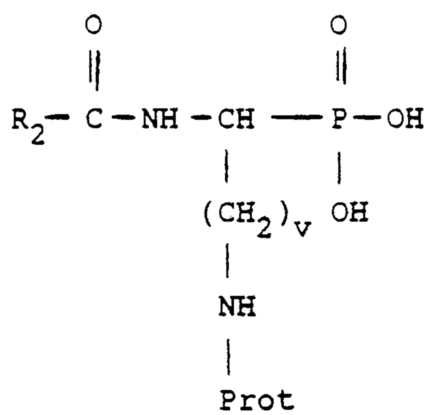

wherein Prot is an amino protecting group such as benzyloxycarbonyl, t-butoxycarbonyl, etc. with a hydroxyacyl amino or imino acid ester of the formula

(III)

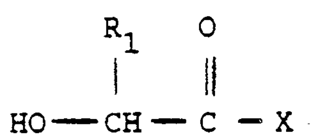

wherein $R_6$ (in the definition of X) is an easily removable ester protecting group, i.e., benzyl.

The above described reaction is preferably performed in the presence of a coupling agent such as dicyclohexylcarbodiimide. Removal of the N-protecting group and the $R_6$ ester group by conventional means such as hydrogenation when Prot is benzyloxycarbonyl and $R_6$ is benzyl yields the desired products of formula I in acid form.

The phosphonic acid of formula II can be prepared as follows. An aldehyde of the formula

(IV)

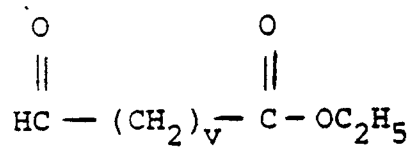

is reacted with triphenylphosphite and the amine (V)

Prot-$NH_2$

wherein Prot is preferably benzyloxycarbonyl to give the phosphonate of the formula

(VI)

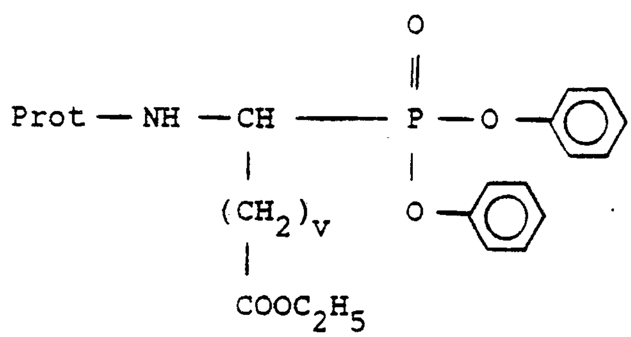

The diphenylphosphonate of formula VI is treated with sodium metal in the presence of ethanol to give the corresponding diethylphosphonate followed by treatment with lithium hydroxide to give the diethylphosphonate of the formula

(VII)

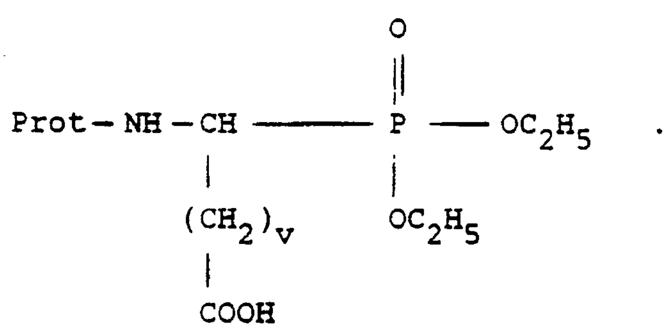

Removal of the N-protecting group and reaction with the acid chloride of the formula

(VIII)

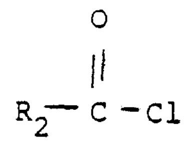

gives the diethylphosphonate of the formula

(IX)

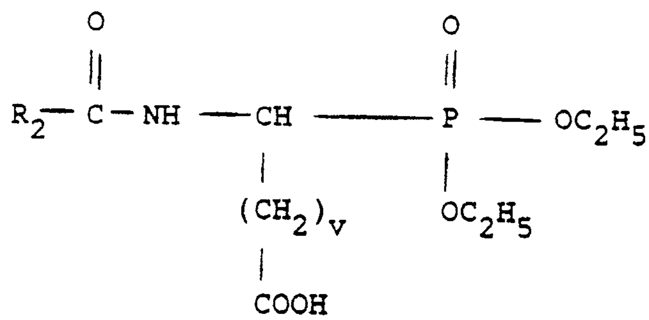

The diethylphosphonate of formula IX is treated with an activating agent such as carbonyldiimidazole and trimethylsilylazide. A solution of the resulting crude azide product is heated and reacted so as to introduce the N-protecting group, for example reacted with benzyl alcohol where Prot is benzyloxycarbonyl, to give the diethylphosphonate of the formula

(X)

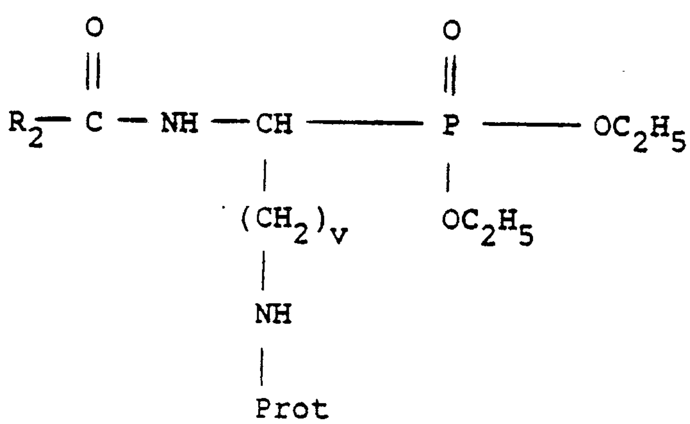

Treatment of the diethylphosphate of formula X with bromotrimethylsilane gives the phosphonic acid of formula II.

The hydroxyacyl amino or imino acid ester of formula III can be prepared by treating the carboxylic acid of the formula

(XI)

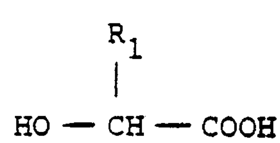

with the amino or imino acid ester of the formula (XII)

H—X

wherein $R_6$ in the definition of X is an easily removable ester protecting group such as benzyl. Preferably, the hydrochloride salt of the ester of formula XII is employed and the reaction is performed in the presence of triethylamine and dicyclohexylcarbodiimide.

In the above reactions if any or all of $R_1$, $R_2$ and $R_{21}$ are

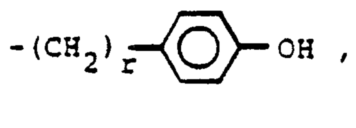 ,

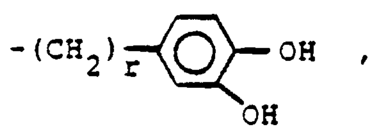 ,

$-(CH_2)_r-NH_2$, 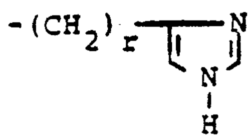 , $-(CH_2)_r-SH$ or

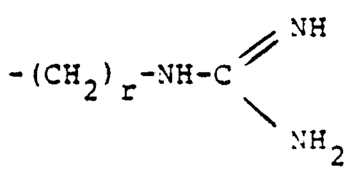

then the hydroxyl, amino, imidazolyl, mercaptan, or guanidinyl function should be protected during the coupling reaction. Suitable protecting groups include benzyloxycarbonyl, t-butoxycarbonyl, trifluoroacetyl, benzyl, benzhydryl, trityl, phthalidyl, etc., and nitro in the case of guanidinyl. The protecting group is removed by hydrogenation, treatment with acid, or other known methods following completion of the reaction.

The ester products of formula I wherein $R_6$ is

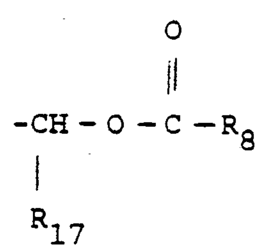

can be obtained by employing the hydroxyacyl amino or imino acid of formula III in the above reactions with the ester group already in place.

The ester products of formula I wherein $R_6$ is

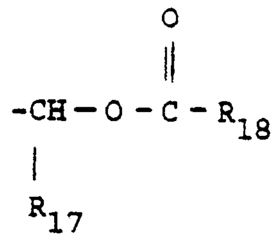

can also be obtained by treating the product of formula I wherein $R_6$ is hydrogen with a molar equivalent of the compound of the formula

(XIII)

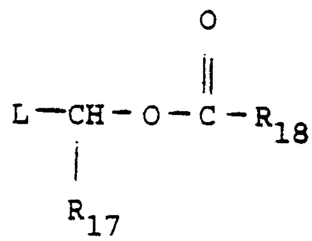

wherein L is leaving group such as chlorine, bromine, tolylsulfonyloxy, etc. The diester products wherein $R_3$ and $R_6$ are the same and are both

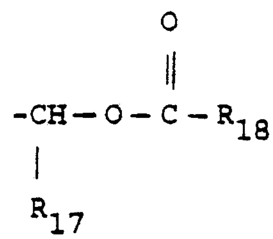

can be obtained by treating the product of formula I wherein $R_3$ and $R_6$ are both hydrogen with two or more equivalents of the compound of formula XIII.

The ester products of formula I wherein $R_3$ is lower alkyl or

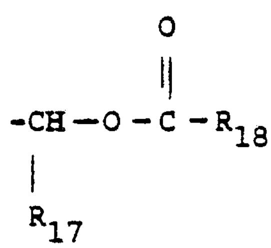

and $R_6$ is hydrogen can be obtained by treating the product of formula I wherein $R_3$ is hydrogen or an alkali metal salt and $R_6$ is benzyl or benzhydryl with the compound of formula XIII. Removal of the $R_6$ ester group such as by hydrogenation yields the desired monoester products.

Preferred compounds of this invention with respect to the amino or imino acid part of the structure are those wherein

X is

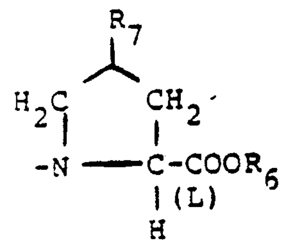 ,

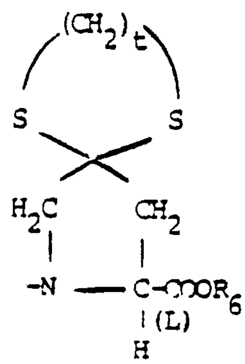 ,

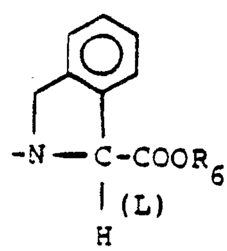 ,

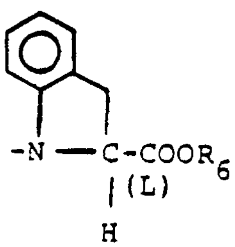 ,

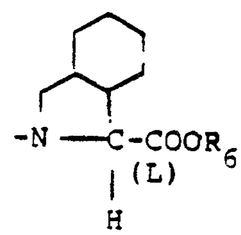 ,

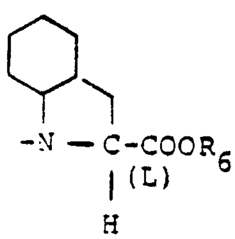 ,

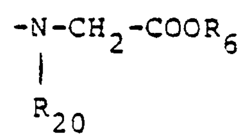 ,

or 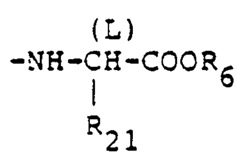 .

$R_7$ is hydrogen, hydroxy, chloro, fluoro, lower alkyl of 1 to 4 carbons, cyclohexyl, amino, -O-lower alkyl wherein lower alkyl is of 1 to 4 carbons, -S-lower alkyl wherein lower alkyl is of 1 to 4 carbons,

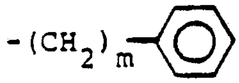, 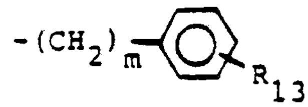,

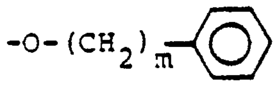, 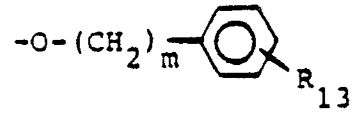,

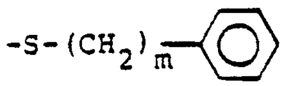, or 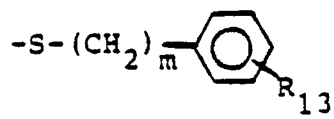.

m is zero, one or two.
$R_{13}$ is methyl, methoxy, chloro, fluoro, bromo, methylthio, or hydroxy.
t is 2 or 3.
$R_{21}$ is hydrogen, straight or branched chain lower alkyl of 1 to 4 carbons,

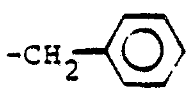, 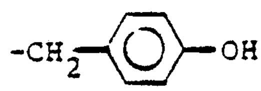,

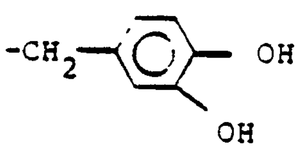, 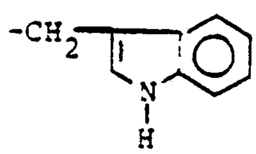,

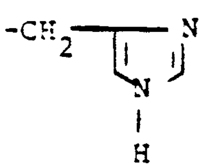, $-(CH_2)_4-NH_2$,

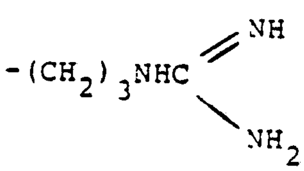, 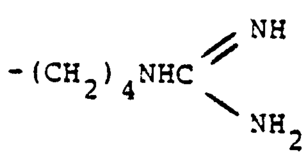,

$-CH_2-SH$, $-CH_2-S-CH_3$, 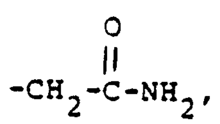, or 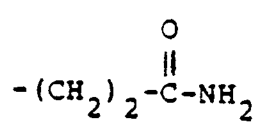.

$R_{20}$ is

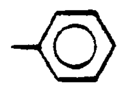 , 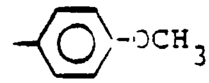 , or

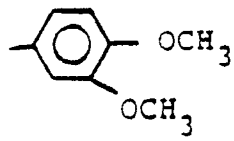 .

$R_6$ is hydrogen, sodium ion, potassium ion, calcium ion, lithium ion, or

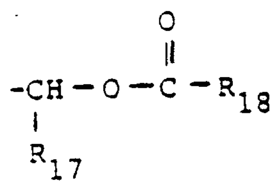 .

$R_{17}$ is hydrogen, straight or branched chain lower alkyl of 1 to 4 carbons, cyclohexyl, or phenyl.

$R_{18}$ is hydrogen or straight or branched chain lower alkyl of 1 to 4 carbons.

Most preferred are those wherein:

X is

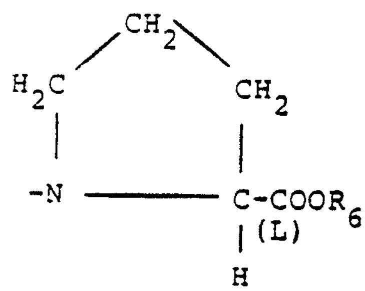 .

$R_6$ is hydrogen, sodium ion, potassium ion, calcium ion, or lithium ion.

Preferred compounds of this invention with respect to the phosphonate part of the structures are those wherein:

$R_1$ is straight or branched chain lower alkyl of 1 to 4 carbons, $-(CH_2)_r-NH_2$, or

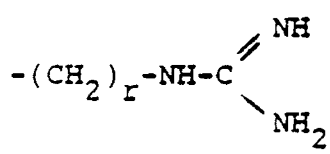

wherein r is an integer from 3 to 5.

$R_2$ is

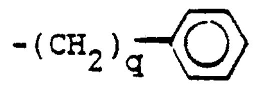,

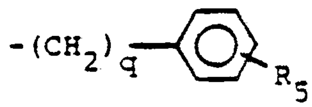,
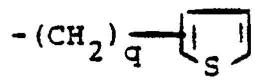,

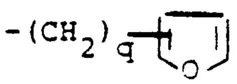, or
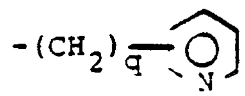.

q is zero, one, or two.

$R_5$ is methyl, methoxy, methylthio, chloro, bromo, fluoro, or hydroxy.

$R_3$ is hydrogen, sodium ion, potassium ion, calcium ion, lithium ion, or

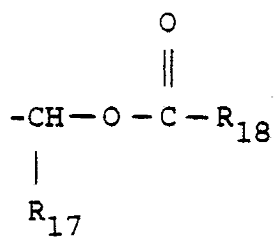.

$R_{17}$ is hydrogen, straight or branched chain lower alkyl of 1 to 4 carbons, cyclohexyl, or phenyl.

$R_{18}$ is hydrogen or straight or branched chain lower alkyl of 1 to 4 carbons.

Most preferred are those wherein:

$R_1$ is $-CH_3$ or $-(CH_2)_4-NH_2$.

$R_2$ is phenyl.

V is 4.

$R_3$ is hydrogen, sodium ion, potassium ion, calcium ion, or lithium ion.

The compounds of formula I wherein at least one of $R_3$ and $R_6$ is hydrogen form salts with a variety of inorganic or organic bases. The nontoxic, pharmaceutically acceptable salts are preferred, although other salts are also useful in isolating or purifying the product. Such pharmaceutically acceptable salts include alkali metal salts such as sodium, potassium or lithium, alklaline earth metal salts such as calcium or magnesium, and salts derived from amino acids such as arginine, lysine, etc. The salts are obtained by reacting the acid form of the compound with an equivalent of the base supplying the desired ion in a medium in which the salt precipitates or in aqueous medium and then lyophilizing.

As shown above, the amino or imino acid or ester portion of the molecule of the products of formula I as represented by X is in the L-configuration. Also, the products of formula I wherein $R_1$ is other than hydrogen contain two asymmetric centers in the phosphonate portion of the molecule as represented by the * in formula I. Additional asymmetric centers are present in the ester products when $R_{17}$ is other than hydrogen. Thus, the compounds of formula I can exist in diastereomeric forms or in mixtures thereof. The above described processes can utilize racemates, enantiomers or diastereomers as starting materials. When diastereomeric products are prepared, they can be separated by conventional chromatographic or fractional crystallization methods.

The products of formula I wherein the imino acid ring is monosubstituted give rise to cis-trans isomerism. The configuration of the final product will depend upon the configuration of the $R_7$, $R_8$ and $R_9$ substituent in the starting material of formula III.

The compounds of formula I, and the pharmaceutically acceptable salts thereof, are hypotensive agents. They inhibit the conversion of the decapeptide angiotensin I to angiotensin II and, therefore, are useful in reducing or relieving angiotensin related hypertension. The action of the enzyme renin on

angiotensinogen, a pseudoglobulin in blood plasma, produces angiotension I. Angiotension I is converted by angiotension converting enzyme (ACE) to angiotension II. The latter is an active pressor substance which has been implicated as the causative agent in several forms of hypertension in various mammalian species, e.g., humans. The compounds of this invention intervene in the angiotensionogen → (renin) → angiotension I → angiotension II sequence by inhibiting angiotension converting enzyme and reducing or eliminating the formulation of the pressor substance angiotension II. Thus by the administration of a composition containing one (or a combination) of the compounds of this invention, angiotension dependent hypertension in a species of mammal (e.g., humans) suffering therefrom is alleviated. A single dose, or preferably two to four divided daily doses, provided on a basis of about 0.1 to 100 mg., preferably about 1 to 50 mg., per kg. of body weight per day is appropriate to reduce blood pressure. The substance is preferably administered orally but parenteral routes such as the subcutaneous, intramuscular, intravenous or intraperitoneal routes can also be employed.

The compounds of this invention can also be formulated in combination with a diuretic for the treatment of hypertension. A combination product comprising a compound of this invention and a diuretic can be administered in an effective amount which comprises a total daily dosage of about 30 to 600 mg., preferably about 30 to 330 mg. of a compound of this invention, and about 15 to 300 mg., preferably about 15 to 200 mg. of the diuretic, to a mammalian species in need thereof. Exemplary of the diuretics contemplated for use in combination of this invention are the thiazide diuretics, e.g., chlorothiazide, hydrochlorothiazide, flumethiazide, hydroflumethiazide, bendroflumethiazide, methyclothiazide, trichloromethiazide, polythiazide or benzthiazide as well as ethacrynic acid, ticrynafen, chlorthalidone, furosemide, musolimine, bumetanide, triamterene, amiloride and spironolactone and salts of such compounds.

The compounds of formula I can be formulated for use in the reduction of blood pressure in compositions such as tablets, capsules or elixirs for oral administration, or in sterile solutions or suspensions for parenteral administration. About 10 to 500 mg. of a compound of formula I is compounded with physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

The compounds of formula I wherein X is

$$-NH-\underset{R_{21}}{\underset{|}{CH}}-COOR_6$$

also possess enkephalinase inhibition activity and are useful as analgesic agents. Thus, by the administration of a composition containing one or a combination of such compounds of formula I or a pharmaceutically acceptable salt thereof, pain is alleviated in the mammalian host. A single dose, or preferably two to four divided daily doses, provided on a basis of about 0.1 to about 100 mg. per kilogram of body weight per day, preferably about 1 to about 50 mg. per kilogram per day, produces the desired analgesic activity. The composition is preferably administered orally but parenteral routes such as subcutaneous can also be employed.

The following examples are illustrative of the invention. Temperatures are given in degrees centigrade. HP-20 refers to a porous crosslinked polystyrene-divinyl benzene polymer resin.

## Example 1

### (S)-1-[2-[[[5-Amino-1-(benzoylamino)pentyl]hydroxyphosphinyl]oxy]-1-oxopropyl]-L-proline, dilithium salt

a) 6-Hydroxyhexanoic acid, ethyl ester

Sodium metal (414 mg., 18 mmole, 0.1 eq.) is dissolved with stirring in absolute ethanol (80 ml.) and the clear solution is treated with freshly distilled ε-caprolactone (20 g., 175 mmole). After 15 minutes the reaction is quenched with glacial acetic acid (1.03 ml., 18 mmole) and concentated *in vacuo*. The oily residue is taken up in methylene chloride, washed with water (twice) and brine, dried over anhydrous sodium sulfate, and evaporated to give 25 g. of 6-hydroxyhexanoic acid, ethyl ester as a light yellow oil. TLC (silica gel; hexane:acetone, 7:3) $R_f$ = 0.14.

b) 6-Oxohexanoic acid, ethyl ester

A mixture of Dess-Martin periodinane (Aldrich, 18.2 g., 43 mmole, 1.2 eq.), t-butanol (4.1 ml., 43 mmole, 1.2 eq.) and dry methylene chloride (100 ml.) is stirred for 15 minutes and then treated with 6-hydroxyhexanoic acid, ethyl ester (6.0 g., 37 mmole) in dry methylene chloride (20 ml.). After stirring for 20 minutes, the suspension is diluted with ether (200 ml.), poured into an aqueous solution of sodium thiosulfate ($Na_2S_2O_3 \cdot 5H_2O$, 71.2 g., 7.0 eq.) in 1N sodium bicarbonate (100 ml.) and stirred vigorously until all the solids dissolve (about 5 minutes). The aqueous layer is discarded and the organic phase is washed successively with 1N sodium bicarbonate, water, and brine, dried over anhydrous magnesium sulfate, and evaporated to give 6.1 g. of 6-oxo-hexanoic acid, ethyl ester as a light yellow oil.

c) 6-[[(Phenylmethoxy)carbonyl]amino]-6-(diphenoxyphosphinyl)hexanoic acid, ethyl ester

Glacial acetic acid (12 ml.) is added to a mixture of benzyl carbamate (5.75 g., 38.0 mmole, 1 eq.), triphenylphosphite (10.0 ml., 38 mmole), and 6-oxohexanoic acid, ethyl ester (6.0 g., 38.0 mmole). The viscous solution is stirred for 3 hours under argon and the acetic acid is removed in vacuo (60°, water bath) to give a crude yellow syrup. This material is purified by flash chromatography (LPS-1 silica gel, 1 kg.) eluting with hexane:methylene chloride:acetone (8:1:1). The product containing fractions are evaporated to give 4.966 g. of 6-[[(phenylmethoxy)carbonyl]amino]-6-(diphenoxyphosphinyl)hexanoic acid, ethyl ester as a viscous yellow oil. TLC (silica gel; hexane: ethyl acetate, 2:3) $R_f$ = 0.75.

d) 6-[[(Phenylmethoxy)carbonyl]amino]-6-(diethoxyphosphinyl)hexanoic acid, ethyl ester

Sodium metal (415 mg., 18.04 mmole, 2.1 eq.) is dissolved with stirring in absolute ethanol (20 ml.) and the clear solution is treated with a solution of 6-[[(phenylmethoxy)carbonyl]amino]-6-(diphenoxyphosphinyl)-hexanoic acid, ethyl ester (4.513 g., 8.59 mmole) in absolute ethanol (20 ml.). After stirring for 30 minutes at room temperature, the yellow mixture is evaporated in vacuo. The residue is taken up in ethyl acetate and washed with water and brine, dried over anhydrous sodium sulfate, and evaporated to an oil. This crude oil is purified by flash chromatography on silica gel (LPS-1, 25 g.) eluting with ethyl acetate:hexane (6:4). Product fractions are evaporated to give 2.936 g. of 6-[[(phenylmethoxy)carbonyl]amino]-6-(diethoxyphosphinyl)hexanoic acid, ethyl ester as a colorless viscous oil. TLC (silica gel; ethyl acetate:hexane, 3:2) $R_f$ = 0.20.

e) 6-[[(Phenylmethoxy)carbonyl]amino]-6-(diethoxyphosphinyl)hexanoic acid

A mixture of 6-[[(phenylmethoxy)carbonyl]amino]-6-(diethoxyphosphinyl)hexanoic acid, ethyl ester (2.318 g., 5.25 mmole) in dioxane (15 ml.) is treated with 1N lithium hydroxide (6.83 ml., 6.83 mmole 1.3 eq.) and the mixture is stirred for 3 hours at room temperature under argon. The mixture is acidified to pH 1 with 6N hydrochloric acid, extracted with ethyl acetate, and the organic phase is washed with water and brine, dried over anhydrous sodium sulfate, and evaporated to give 2.18 g. of 6-[[(phenylmethoxy)carbonyl]-amino]-6-(diethoxyphosphinyl)hexanoic acid as a clear, viscous oil. TLC (silica gel; methylene chloride:methanol, 9:1) $R_f$ = 0.51.

f) 6-Amino-6-(diethoxyphosphinyl)hexanoic acid

20% Palladium hydroxide on carbon catalyst (560 mg., 15% by weight) is added to an argon purged solution of 6-[[(phenylmethoxy)carbonyl]amino]-6-(diethoxyphosphinyl)hexanoic acid (3.73g., 8.98 mmole) in methanol (25 ml.) and the black suspension is stirred under hydrogen for 2 hours. The catalyst is removed by filtration through dry, packed Celite and the filtrate is evaporated to give 2.342 g. of 6-amino-6-(diethoxyphosphinyl)hexanoic acid as a colorless, viscous oil. TLC (silica gel; methylene chloride: methanol, 9:1) $R_f$ = 0.10.

g) 6-(Benzoylamino)-6-(diethoxyphosphinyl)hexanoic acid

A mixture of 6-amino-6-(diethoxyphosphinyl)hexanoic acid (2.162g., 8.09 mmole) in dry acetonitrile (25 ml.) is treated with bis(trimethylsilyl) trifluoroacetamide (6.5 ml., 24.3 mmole, 3.0 eq.) and the clear solution is stirred under argon at room temperature for 45 minutes. Benzoyl chloride (1.13 ml., 9.7 mmole, 1.2 eq.), dimethylaminopyridine (148 mg., 1.21 mmole, 0.15 eq.), and triethylamine (0.56 ml., 4.05 mmole, 0.5 eq.)

are added and the mixture is stirred for 6 hours. The mixture is evaporated in vacuo taken up in acetonitrile (10 ml.), treated with water (4 ml.), stirred for 15 minutes, and then evaporated. The residue is dissolved in ethyl acetate and washed successively with 5% potassium bisulfate and brine, dried over anhydrous sodium sulfate, and evaporated to a yellow oil. This crude oil is purified by flash chromatography (LPS-1 silica gel, 120 g.) eluting with methylene chloride:methanol:acetic acid (60:1:1). Product fractions are evaporated and residual acetic acid is removed by azeotroping several times with toluene to give 1.573 g. of 6-(benzoylamino)-6-(diethoxyphosphinyl)hexanoic acid as a clear viscous oil. TLC (silica gel; methylene chloride:methanol:acetic acid) $R_f$ = 0.62.

h) [1-(Benzoylamino)-5-[[(phenylmethoxy)carbonyl]amino]pentyl]phosphonic acid, diethyl ester

A solution of 6-(benzoylamino)-6-(diethoxyphosphinyl)hexanoic acid (1.334 g., 3.59 mmole) in toluene (10 ml.) is treated with carbonyldiimidazole (700 mg., 4.31 mmole, 1.2 eq.) and stirred for 1 hour at room temperature. It is then treated with trimethylsilylazide (0.62 ml., 4.67 mmole, 1.3 eq.) And stirred for an additional 1.5 hours. The reaction mixture is partitioned between 5% potassium bisulfate and ethyl acetate. The organic phase is washed with water and brine, dried over anhydrous sodium sulfate, and evaporated to an oil. This crude acyl azide is taken up in toluene (10 ml.), heated at 90° (oil bath) for 2 hours, evaporated, taken up in toluene (1 ml.), treated with benzyl alcohol (1.11 ml., 10.77 mmole, 3 eq.) and heated overnight at 90° under argon. The mixture is evaporated to an oil which is purified by flash chromatography (LPS-1 silica gel, 100 g.) eluting with hexane:acetone (3:2). Product fractions are evaporated to give 1.313 g. of [1-(benzoylamino)-5-[[(phenylmethoxy)carbonyl]amino]pentyl]phosphonic acid, diethyl ester as a colorless, viscous oil. TLC (silica gel, methylene chloride:acetone, 2:1) $R_f$ = 0.35.

i) [1-(Benzoylamino)-5-[[(phenylmethoxy)carbonyl]amino]pentyl]phosphonic acid

A solution of the diethyl ester product from part (h) (1.313 g., 2.76 mmole) in dry methylene chloride (10 ml.) is treated with bromotrimethylsilane (0.80 ml., 6.07 mmole, 2.2 eq.) and the clear solution is stirred overnight under argon. The orange mixture is evaporated in vacuo, taken up in dioxane (10 ml.), treated with water (3 ml.), stirred for 15 minutes and then evaporated in vacuo. Residual water is removed by azeotroping with acetonitrile (3 times) to give 1.156 g. of [1-(benzoylamino)-5-[[(phenylmethoxy)carbonyl]-amino]pentyl]phosphonic acid as an orange foam. TLC (silica gel; isopropanol:ammonia:water, 7:2:1) Rf = 0.31.

j) 1-[(S)-2-Hydroxy-1-oxopropyl]-L-proline, phenylmethyl ester

A mixture of sodium lactate (1.7 g., 15.0 mmole), diphenylphosphorylazide (3.6 ml., 1.1 eq.), and dry dimethylformamide (30 ml.) at 0° (ice-bath) in an argon atmosphere is treated with triethylamine (2.1 ml., 1.0 eq.) and L-proline, phenylmethyl ester, monohydrochloride salt (3.6 g., 15.0 mmole). After 24 hours, the reaction mixture is partitioned between ethyl acetate and water. The aqueous phase is back extracted, the organic extracts are combined, washed with 5% potassium bisulfate, brine, and evaporated. The residue (5 g.) is chromatographed on silica (130 g.) eluting with (1:1, ethyl acetate/hexane) to give 2.5 g. of 1-[(S)-2-hydroxy-1-oxopropyl]-L-proline, phenylmethyl ester as a white crystalline solid after evaporation; m.p. 86-88° (isopropyl ether). TLC (silica gel; ethyl acetate) $R_f$ = 0.4.

Anal. calc'd. for $C_{15}H_{19}NO_4$:
C, 64.97; H, 6.91; N, 5.05
Found: C, 64.70; H, 6.85; N, 5.02.

k) (S)-1-[2-[[[5-[[(Phenylmethoxv)carbonyl]amino]-1-(benzoylamino)pentyl]hydroxyphosphinyl]oxy]-1-oxopropyl]-L-proline, phenylmethyl ester

A mixture of 1-[(S)-2-hydroxy-1-oxopropyl]-L-proline, phenylmethyl ester (416 mg., 1.50 mmole, 1 eq.) and [1-(benzoylamino)-5-[[(phenylmethoxy)carbonyl]amino]pentyl]phosphonic acid (629 mg., 1.50 mmole, 1 eq.) in dry tetrahydrofuran (10 ml.) at 0° (ice bath) is treated with dicyclohexylcarbodiimide (371 mg., 1.8 mmole, 1.2 eq.) and the suspension is stirred at 0° for 1 hour and then 4.5 hours at room temperature. The mixture is diluted with 1N hydrochloric acid, filtered, and the filtrate is extracted with ethyl acetate. The organic phase is washed with saturated sodium bicarbonate and brine, dried over anhydrous sodium sulfate, and evaporated to give 830 mg. of (S)-1-[2-[[[5-[[(phenylmethoxy)carbonyl]amino]-1-(benzoylamino)pentyl]-hydroxyphosphinyl]oxy]-1-oxopropyl]-L-proline, phenylmethyl ester as an oil. TLC (silica gel; methylene

chloride:methanol:acetic acid, 20:1:1) $R_f$ = 0.25.

l) (S)-1-[2-[[[5-Amino-1-(benzoylamino)pentyl]hydroxyphosphinyl]oxy]-1-oxopropyl]-L-proline, dilithium salt

20% Palladium hydroxide on carbon catalyst (125 mg., 15% by weight) is added to an argon purged solution of thje ester product from part (k) (830 mg.) in methanol (10 ml.) and the black suspension is stirred under hydrogen for 2.5 hours. The catalyst is removed by filtration through dry, packed Celite and the filtrate is evaporated to a white foam. The crude solid is dissolved in 1N lithium hydroxide (4.0 ml.), diluted with water, filtered through a polycarbonate membrane and prefilter, concentrated to a small volume, and then chromatographed on HP-20 resin eluting with water (100%) → acetonitrile (100%) linear gradient system. Product fractions are evaporated, taken up in water (50 ml.), filtered through a polycarbonate membrane, frozen, and lyophilized to give 256 mg. of (S)-1-[2-[[[5-amino-1-(benzoylamino)pentyl]-hydroxyphosphinyl]oxy]-1-oxopropyl]-L-proline, dilithium salt as a white solid; $[\alpha]_D$ = -37.2° (c = 0.5, methanol). TLC (silica gel; isopropanol:ammonia:water, 7:2:1) $R_f$ = 0.21 and 0.29 (two diastereomers).
Anal. calc'd. for $C_{20}H_{28}N_3O_7P$ • 2Li • 2.5 $H_2O$:
C, 46.88; H, 6.49; N, 8.20; P, 6.04
Found: C, 46.84; H, 6.46; N, 7.99; P, 6.00.

Example 2

1-[(S)-6-Amino-2-[[[5-amino-1-(benzoylamino)pentyl]hydroxyphosphinyl]oxy]-1-oxohexyl]-L-proline, dilithium salt

a) (S)-6-Amino-2-hydroxyhexanoic acid

An aqueous solution of L-lysine, monohydrochloride (18.3 g., 0.1 mole) is passed through an AG 3-X4A (100 - 200 mesh) ion exchange column (OH form, 500 ml. Bed volume) eluting with water. The ninhydrin positive fractions are combined, acidified with 2M (4N) sulfuric acid (100 ml., 0.2 mole) and evaporated to dryness.

The crude L-lysine, disulfuric acid salt is taken up in 10 % sulfuric acid (250 ml.) And treated dropwise with a solution of sodium nitrite (25.9 g., 0.36 mole) in water (100 ml.) At 45 - 50° (bath temperature) over a period of 2 hours. When the addition is complete, the mixture is stirred at 45 - 50° for an additional 4.5 hours, the excess nitrous acid decomposed with urea and the mixture is poured onto an AG-50-X8 ion exchange column ($H^+$ form, 200 ml. bed volume). The column is eluted with water and then aqueous ammonia (concentrated ammonia-water, 1:3) to elute the product. The ninhydrin positive fractions are combined and evaporated to give a pink semisolid which is recrystallized from water-ethanol to give 8.20 g. of (S)-6-amino-2-hydroxyhexanoic acid as white crystals; m.p. 197-199°; $[\alpha]_D^{22}$ = -12.2° (c = 1.2, water). TLC(silica gel; isopropanol: concentrated ammonia:water, 7:2:1) $R_f$ = 0.16 (contains trace of lysine, $R_f$ = 0.22).

b) (S)-6-[[(Phenylmethoxy)carbonyl]amino] -2-hydroxyhexanoic acid

A solution of (S)-6-amino-2-hydroxyhexanoic acid (7.5 g., 51.0 mmole) in 1N sodium hydroxide solution (50 ml.) at 0° (ice-bath) is adjusted to pH 10.0 with concentrated hydrochloric acid and treated with benzyl chloroformate (8.4 ml., 95%, 55.9 mmole) in approximately 1 ml. portions at 15 minute intervals. Throughout the reaction, the pH is maintained at pH 9.8 - 10.2 by the addition of 1N sodium hydroxide solution. When the addition is complete and the pH stabilized, the mixture is stirred at pH 10, 0°, for an additional 45 minutes, and then washed with one portion of ethyl ether. The aqueous solution is acidified to pH 1 with concentrated hydrochloric acid and extracted with ethyl acetate. The ethyl acetate extract is washed with saturated sodium chloride solution, dried over sodium sulfate, and evaporated. The residue is crystallized from isopropyl ether to give 13.5 g. of crude product as a white solid. Recrystallization of the crude product from ethyl acetate-hexane gives 11.48 g. of (S)-6-[[(phenylmethoxy)carbonyl]amino]-2-hydroxyhexanoic acid as a white crystalline solid; m.p. 79 - 81°; $[\alpha]_D^{22}$ = +4.5°, $[\alpha]_{365}$ = +26.8° (c = 1.1, chloroform). TLC (silica gel; acetic acid: methanol:methylene chloride, 1:1:20) $R_f$ = 0.19.

c) 1-[(S)-6-[[(Phenylmethoxy)carbonyl]amino]-2-hydroxy-1-oxohexyl]-L-proline, phenylmethyl ester

A mixture of (S)-6-[[(phenylmethoxy)carbonyl]amino]-2-hydroxyhexanoicacid (1.4 g., 5.0 mmole), L-proline, phenylmethyl ester, monohydrochloride (1.33 g., 5.5 mmole), and triethylamine (0.76 ml., 5.5 mmole) in dry tetrahydrofuran (15 ml.) at 0° (ice-bath) is treated with 1-hydroxybenzotriazole hydrate (0.71 g., 5.26 mmole) and dicyclohexylcarbodiimide (1.08 g., 5.23 mmole). The solution is stirred at 0° for 3 hours, then allowed to warm to room temperature and stirred for an additional one hour. The mixture is filtered, diluted with ethyl acetate, and washed successively with 5% potassium bisulfate, saturated sodium bicarbonate, and saturated sodium chloride, dried over sodium sulfate, and evaporated. The residue is taken up in carbon tetrachloride, filtered to remove the last traces of dicyclohexyl urea, and evaporated. The crude product is purified by flash chromatography on silica gel (35 g., Whatman LPS-1) eluting with ethyl acetate-hexane (2:1) to give 2.24 g. of 1-[(S)-6-[[(phenylmethoxy)carbonyl]amino]-2-hydroxy-1-oxohexyl]-L-proline, phenylmethyl ester as a colorless, very viscous oil. TLC (silica gel; methanol:methylene chloride, 5:95) $R_f$ = 0.36.

d) 1[(S)-6-[[(Phenylmethoxy)carbonyl]amino]-2-[[[5-[[(phenylmethoxy)carbonyl]amino]-1-(benzoylamino)-pentyl]hydroxyphosphinyl]oxy]-1-oxohexyl]-L-proline, phenylmethyl ester

A mixture of [1-(benzoylamino)-5-[[(phenylmethoxy)carbonyl]amino]pentyl]phosphonic acid (606 mg., 1.44 mmole, 1 eq.) and 1-[(S)-6-[[(phenylmethoxy)carbonyl]amino]-2-hydroxy-1-oxohexyl]-L-proline, phenylmethyl ester (674 mg., 1.44 mmole, 1 eq.) in dry tetrahydrofuran (15 ml.) at 0° (ice bath) is treated with dicyclohexylcarbodiimide (446 mg., 2.16 mmole, 1.5 eq.) and the white suspension is stirred under argon at 0° for 1 hour and an additional 4.5 hours at room temperature. The suspension is diluted with 1N hydrochloric acid, filtered, and the filtrate is extracted with ethyl acetate. The organic layer is washed with saturated sodium bicarbonate and brine, dried over anhydrous sodium sulfate, and evaporated to give 1.096 g. of 1-[(S)-6-[[(phenylmethoxy)carbonyl]amino]-2-[[[5-[[(phenylmethoxy)carbonyl]amino]-1-(benzoylamino)-pentyl]hydroxyphosphinyl]oxy]-1-oxohexyl]-L-proline, phenylmethyl ester as a viscous oil. TLC (silica gel; methylene chloride: methanol:acetic acid, 20:1:1) $R_f$ = 0.28 and 0.35.

e) 1-[(S)-6-Amino-2-[[[5-amino-1-(benzoylamino)pentyl]hydroxyphosphinyl]oxy]-1-oxohexyl]-L-proline, dilithium salt

20% Palladium hydroxide on carbon catalyst (164 mg., 15% by weight) is added to an argon purged solution of the ester product from part (d) (1.096 g.) in methanol (10 ml.) and the black suspension is stirred under hydrogen for 3 hours. The catalyst is removed by filtration through dry, packed Celite and the filtrate is evaporated to a white foam. The crude product is taken up in 1 N lithium hydroxide (40 ml.), diluted with water, filtered through a polycarbonate membrane and prefilter, concentrated to a few ml. volume, and chromatographed on an HP-20 column eluting with water (100%) → (50:50) water:acetonitrile gradient system. Product fractions are combined, evaporated, taken up in water (50 ml.), filtered through a polycarbonate membrane, frozen, and lyophilized to give 333 mg. of 1-[(S)-6-amino-2-[[[5-amino-1-(benzoylamino)pentyl]hydroxyphosphinyl]oxy]-1-oxohexyl]-L-proline, dilithium salt as a white solid; $[\alpha]_D$ = -31.0° (c = 0.5 methanol). TLC (silica gel; isopropanol:ammonia:water, 7:2:1) $R_f$ = 0.10.

Anal. calc'd. for $C_{23}H_{35}N_4O_7P \cdot 2Li \cdot 1.45\ H_2O$:
C, 50.18; H, 6.94; N, 10.18; P, 5.63
Found: C, 50.20; H, 7.37; N, 9.83; P, 5.50.

Examples 3 - 33

In a similar manner the following products can be obtained.

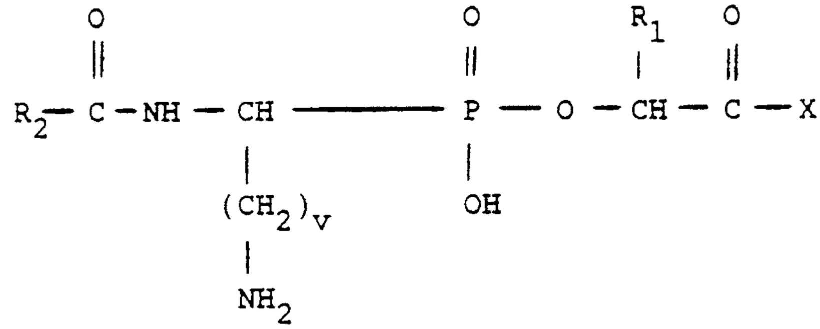

| Example | $R_2$ | v | $R_1$ | X |
|---|---|---|---|---|
| 3 | 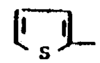 | 3 | -H | 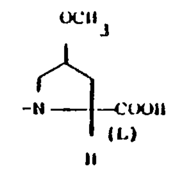 |
| 4 | 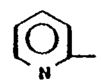 | 5 | $-CH_3$ | 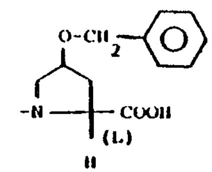 |
| 5 | $H_3C-(H_2C)_5-$ | 4 | 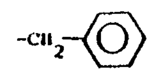 | 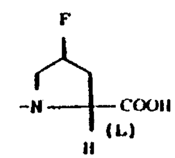 |
| 6 | 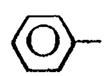 | 4 | $-(CH_2)_4-NH_2$ | 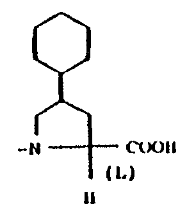 |

| Example | $R_2$ | v | $R_1$ | X |
|---|---|---|---|---|
| 7 | 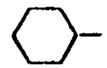 | 4 | $-CH_3$ | 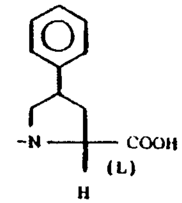 |
| 8 | 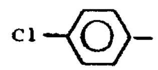 | 3 | 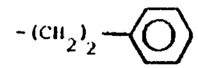 | 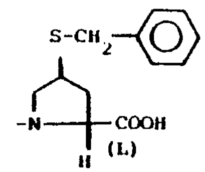 |
| 9 | 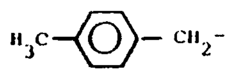 | 5 | $-CH_3$ | 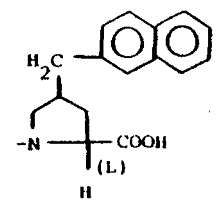 |
| 10 | 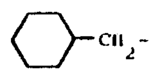 | 4 | 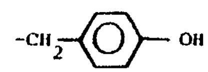 | 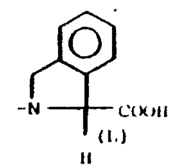 |

| Example | $R_2$ | v | $R_1$ | X |
|---|---|---|---|---|
| 11 | $H_3C-(H_2C)_6-$ | 4 | $-(CH_2)_5-NH_2$ | 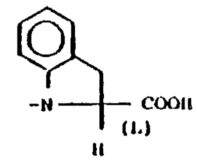 |
| 12 | 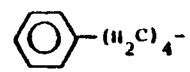 | 4 | 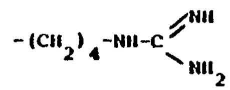 | 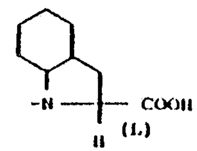 |
| 13 | 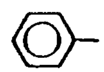 | 5 | 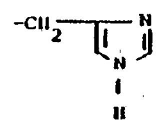 | 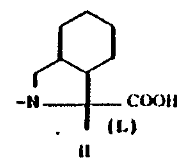 |
| 14 | 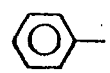 | 3 | 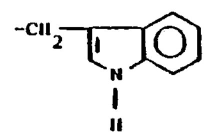 | 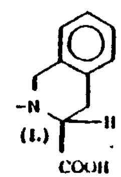 |

| Example | $R_2$ | v | $R_1$ | X |
| --- | --- | --- | --- | --- |
| 15 | 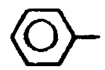 | 4 | $-(CH_2)_4-NH_2$ | 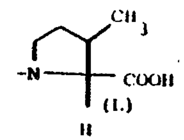 |
| 16 | 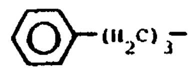 | 4 | $-CH_3$ | 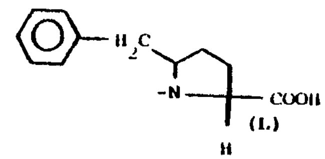 |
| 17 | 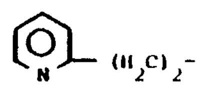 | 4 | 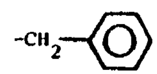 | 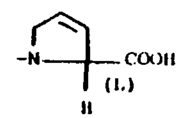 |
| 18 | 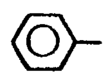 | 4 | $-CH_3$ | 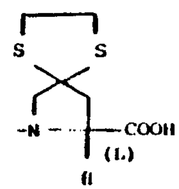 |

| Example | $R_2$ | v | $R_1$ | X |
|---|---|---|---|---|
| 19 | 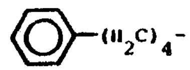 | 4 | $-(CH_2)_4-NH_2$ | 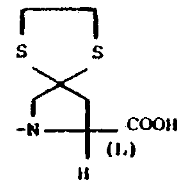 |
| 20 | 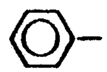 | 4 | $-(CH_2)_2-S-CH_3$ | 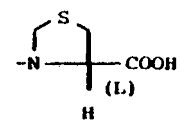 |
| 21 | 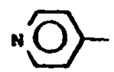 | 3 | $-CH_3$ | 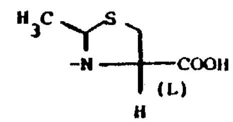 |
| 22 | 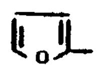 | 5 | $-CH_3$ | 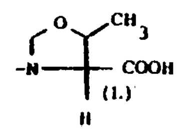 |

| Example | $R_2$ | v | $R_1$ | X |
|---|---|---|---|---|
| 23 | 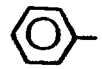 | 4 | $-(CH_2)_3-NH_2$ | 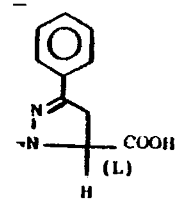 |
| 24 | 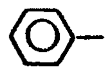 | 4 | $-CH_3$ | 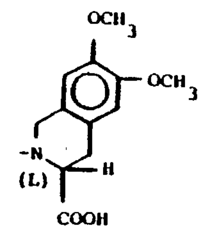 |
| 25 | 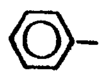 | 4 | $-CH_3$ | 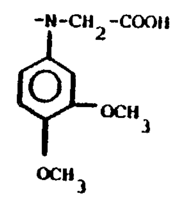 |

| Example | $R_2$ | v | $R_1$ | X |
|---|---|---|---|---|
| 26 | 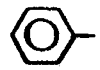 | 3 | $-(CH_2)_4-NH_2$ | 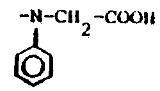 |
| 27 | 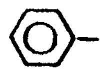 | 4 | $-CH_3$ | (L) 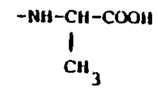 |
| 28 | 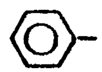 | 5 | $-CH_3$ | (L) 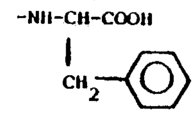 |
| 29 | 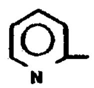 | 4 | $-CH_3$ | (L) 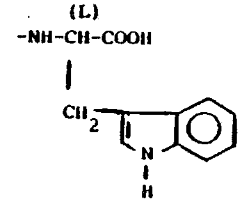 |
| 30 | 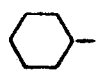 | 4 | $-CH_3$ | 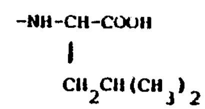 |

| Example | $R_2$ | v | $R_1$ | X |
|---|---|---|---|---|
| 31 | 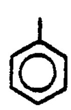 | 4 | $-(CH_2)_4-NH_2$ | 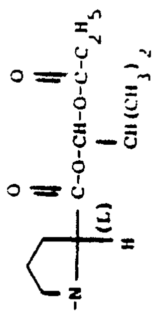 |
| 32 | 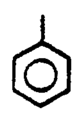 | 3 | $-CH_3$ | 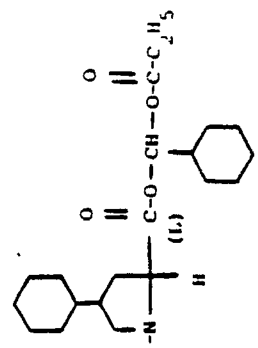 |
| 33 | 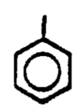 | 5 | $-CH_3$ | 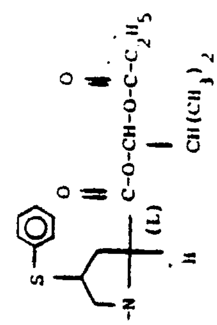 |

Example 34

1000 tablets each containing the following ingredients:

| | |
|---|---|
| (S)-1-[2-[[[5-Amino-1-(benzoylamino)pentyl]hydroxyphosphinyl]oxy]-1-oxopropyl]-L-proline, dilithium salt | 100 mg. |
| Cornstarch | 50 mg. |
| Gelatin | 7.5 mg. |
| Avicel (Microcrystalline cellulose) | 25 mg. |
| Magnesium stearate | 2.5 mg. |
| | 185 mg. |

are prepared from sufficient bulk quantities by mixing the product of Example 1 and cornstarch with and aqueous solution of the gelatin. The mixture is dried and ground to a powder. The Avicel and then the magnesium stearate are admixed with granulation. This mixture is then compressed in a tablet press to form 1000 tablets each containing 100 mg. of active ingredient. This same procedure can be employed to prepare tablets containing 50 mg. of active ingredient.

Similarly, tablets containing 100 mg. of the product of any of Examples 2 to 33 can be prepared.

Example 35

Two piece #1 gelatin capsules are filled with a mixture of the following ingredients:

| | |
|---|---|
| 1-[(S)-6-Amino-2-[[[5-amino-1-(benzoylamino)pentyl]hydroxyphosphinyl]oxy]-1-oxohexyl]-L-proline, dilithium salt | 100 mg. |
| Magnesium stearate | 7 mg. |
| Lactose | 193 mg. |
| | 300 mg. |

In a similar manner capsules containing 100 mg. of the product of any of Examples 1 and 3 to 33 can be prepared.

Example 36

An injectable solution is prepared as follows:

| | |
|---|---|
| (S)-1-[2-[[[5-Amino-1-(benzoylamino)pentyl]hydroxyphosphinyl]oxy]-1-oxopropyl]-L-proline, dilithium salt | 500 g. |
| Methyl paraben | 5 g. |
| Propyl paraben | 1 g. |
| Sodium chloride | 25 g. |
| Water for injection | 5 l. |

The active substance, preservatives and sodium chloride are dissolved in 3 liters of water for injection and then the volume is brought up to 5 liters. The solution is filtered through a sterile filter and aseptically filled into presterilized rubber closures. Each vial contains 5 ml. of solution in a concentration of 100 mg. of active ingredient per ml. of solution for injection.

In a similar manner, an injectable solution containing 100 mg. of active ingredient per ml. of solution can be prepared for the product of any of Examples 2 to 33.

Example 37

1000 tablets each containing the following ingredients:

| | |
|---|---|
| 1-[(S)-6-Amino-2-[[[5-amino-1-(benzoylamino)pentyl]hydroxyphosphinyl]oxy]-1-oxohexyl]-L-proline, dilithium salt | 100 mg. |
| Avicel | 100 mg. |
| Hydrochlorothiazide | 12.5 mg. |
| Lactose | 113 mg. |
| Cornstarch | 17.5 mg. |
| Stearic acid | 7 mg. |
| | 350 mg. |

are prepared from sufficient bulk quantities by slugging the product of Example 2, Avicel, and a portion of the stearic acid. The slugs are ground and passed through a #2 screen, then mixed with the hydrochlorothiazide, lactose, cornstarch, and the remainder of the stearic acid. Ths mixture is compressed into 350 mg. capsule shaped tablets in a tablet press. The tablets are scored for dividing in half.

In a similar manner, tablets can be prepared containing 100 mg. of the products of any of Examples 1 and 3 to 33.

## Claims

**1.** A compound of the formula

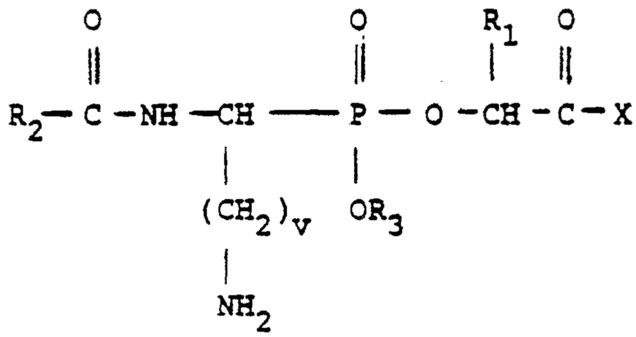

including a pharmaceutically acceptable salt thereof wherein:

$R_1$ is hydrogen, lower alkyl, $-(CH_2)_r-Cl$,

$-(CH_2)_r-Br$, $-(CH_2)_r-F$, $CF_3$, 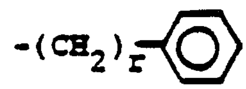 ,

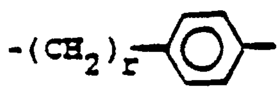 , 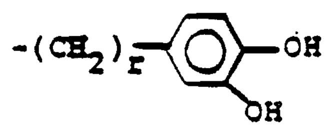 ,

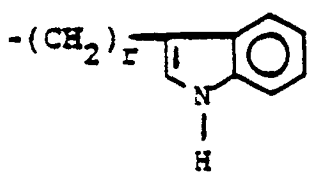 , 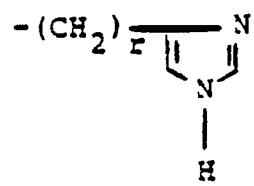 ,

$-(CH_2)_r$-cycloalkyl, $-(CH_2)_r-NH_2$, $-(CH_2)_r-SH$,

$-(CH_2)_r$-S-lower alkyl, 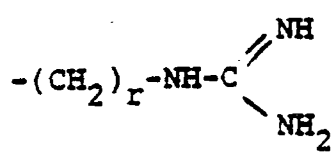 , or

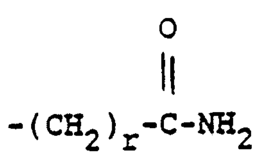 ;

$R_2$ is straight or branched chain alkyl of 1 to 10 carbons, $-(CH_2)_q$-cycloalkyl,

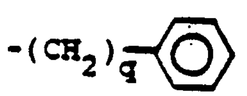 ,

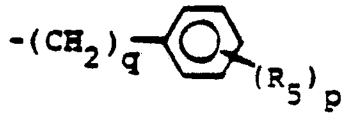 , 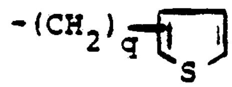 ,

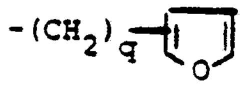 or 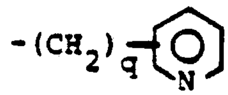 ;

$R_3$ is hydrogen, lower alkyl, benzyl, alkali metal salt ion, alkaline earth metal salt ion, or

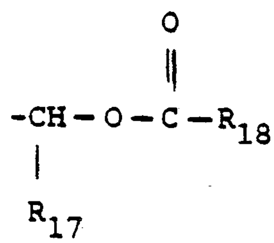 ;

v is an integer from 3 to 5;
r is an integer from 1 to 7;
q is zero or an integer from 1 to 7;
X is an amino or imino acid or ester of the formula

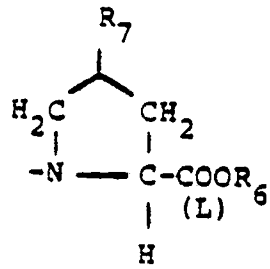 , 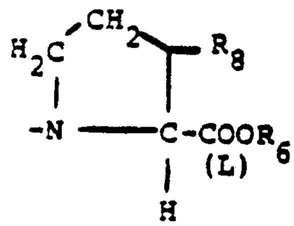 ,

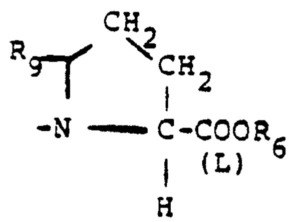 , 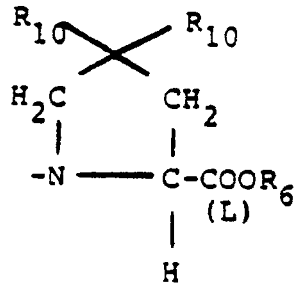 ,

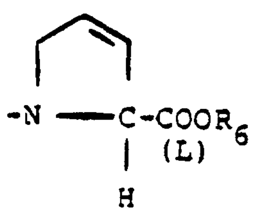

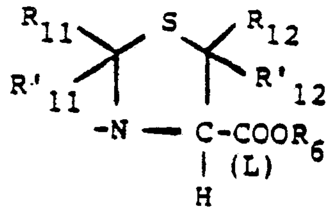 , 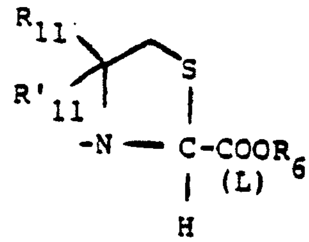 ,

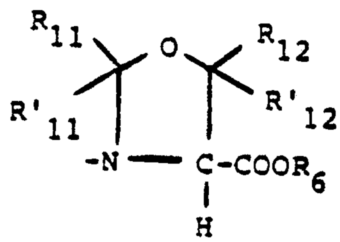

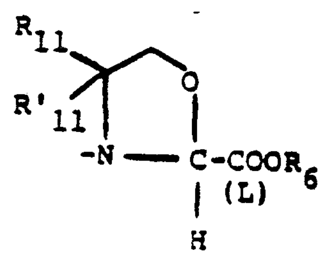

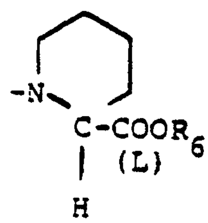 , 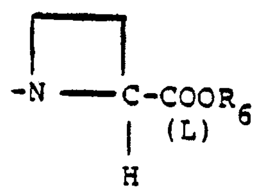 ,

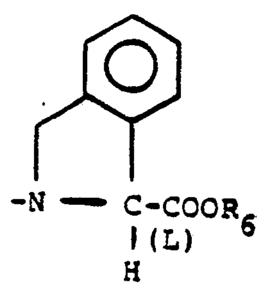 , 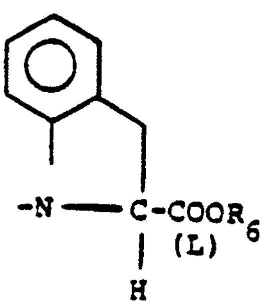 ,

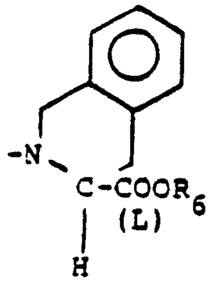 , 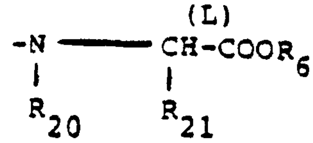

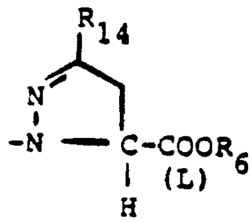 , 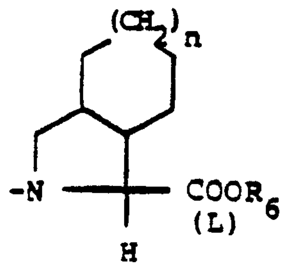 ,

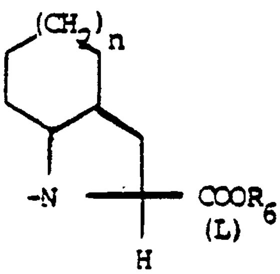 , 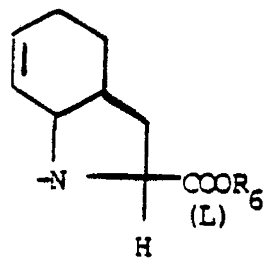 ,

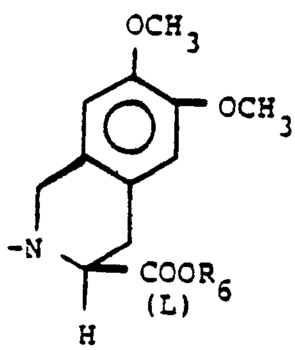 ; 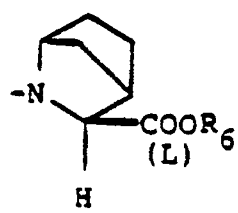 ,

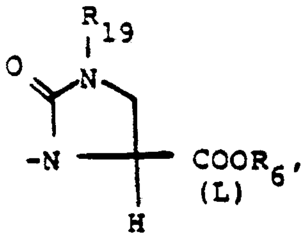 or 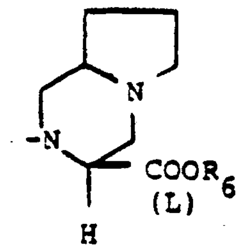 ;

$R_7$ is hydrogen, lower alkyl, halogen, hydroxy,

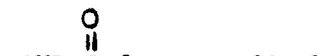 amino, 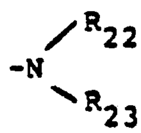 ,

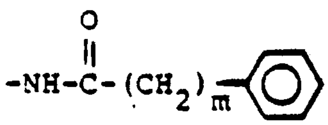 , 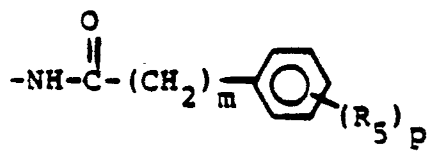 ,

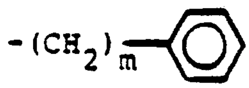 , 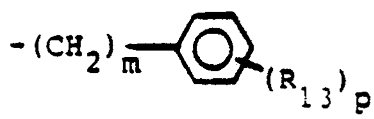 ,

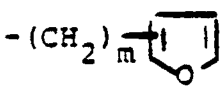 , 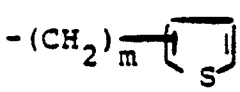 , 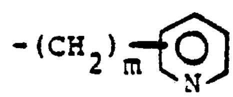 ,

a 1- or 2-naphthyl of the formula

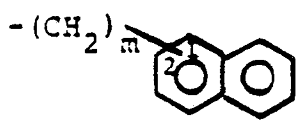 ,

a substituted 1- or 2-naphthyl of the formula

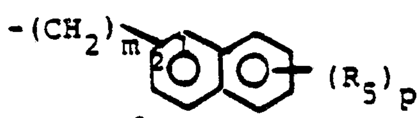 ,

$-(CH_2)_m$-cycloalkyl, 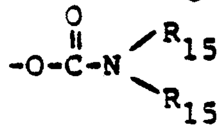 , -O-lower alkyl,

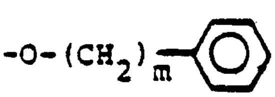 , 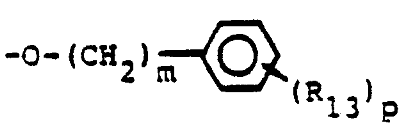 ,

a 1- or 2- naphthyloxy of the formula

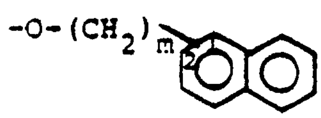,

a substituted 1- or 2-naphthyloxy of the formula

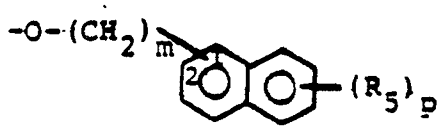,

-S-lower alkyl,

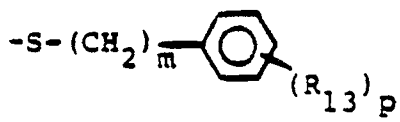,

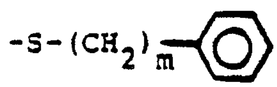,

a 1- or 2-naphthylthio of the formula

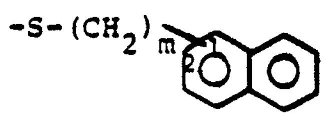,

or a substituted 1- or 2-naphthylthio of the formula

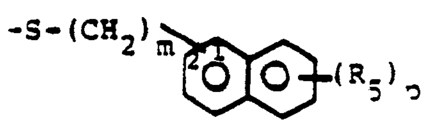;

$R_8$ is lower alkyl, halogen,

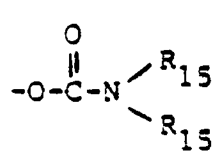,

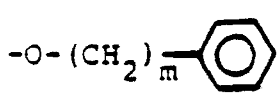, 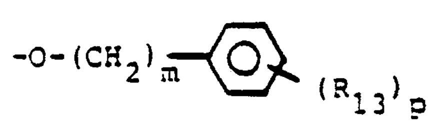,

-O-lower alkyl, a 1- or 2-naphthyloxy of the formula

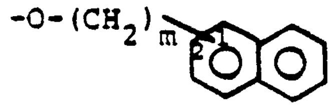,

a substituted 1- or 2-naphthyloxy of the formula

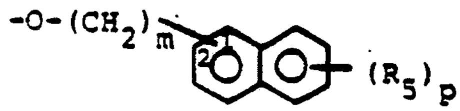,

-S-lower alkyl,

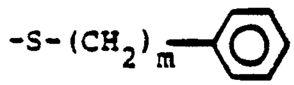,

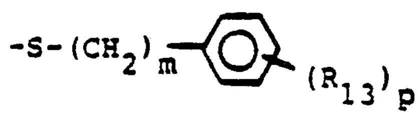,

a 1- or 2-naphthylthio of the formula

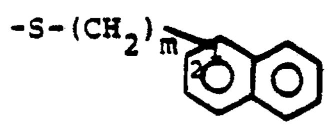,

or a substituted 1- or 2-naphthylthio of the formula

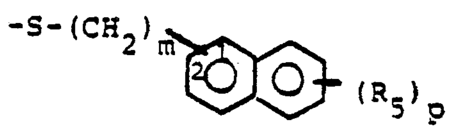;

$R_9$ is lower alkyl, keto,

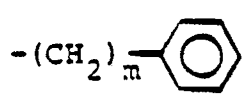,

or

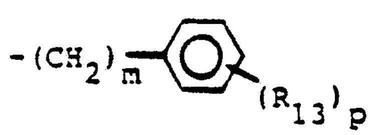;

$R_{10}$ is halogen or Y-$R_{16}$;

$R_{11}$, $R'_{11}$, $R_{12}$ and $R'_{12}$ are independently selected from hydrogen and lower alkyl or $R'_{11}$, $R_{12}$ and $R'_{12}$ hydrogen and $R_{11}$ is

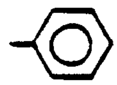 or 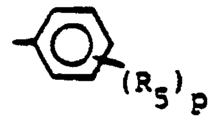 ;

$R_{13}$ is lower alkyl of 1 to 4 carbons, lower alkoxy of 1 to 4 carbons, lower alkylthio of 1 to 4 carbons, chloro, bromo, fluoro, trifluoromethyl, hydroxy, phenyl, phenoxy, phenylthio, or phenylmethyl;

$R_5$ is lower alkyl of 1 to 4 carbons, lower alkoxy of 1 to 4 carbons, lower alkylthio of 1 to 4 carbons, chloro, bromo, fluoro, trifluoromethyl or hydroxy;

m is zero, one, two, three, or four;

p is one, two or three provided that p is more than one only if $R_{13}$ or $R_5$ is methyl, methoxy, chloro, bromo, or fluoro;

$R_{14}$ is hydrogen, lower alkyl,

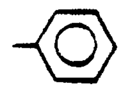 ,

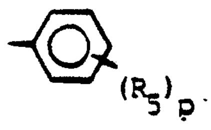 , 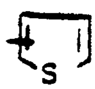 , 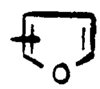 , or 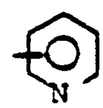 ;

$R_{15}$ is hydrogen or lower alkyl of 1 to 4 carbons;

Y is oxygen or sulfur;

$R_{16}$ is lower alkyl of 1 to 4 carbons,

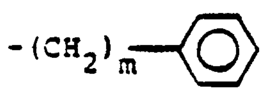 , 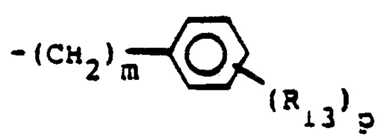 ,

or the $R_{16}$ groups join to complete an unsubstituted 5- or 6-membered ring or said ring in which one or more of the carbons has a lower alkyl of 1 to 4 carbons or a di(lower alkyl of 1 to 4 carbons) substituent;

$R_{17}$ is hydrogen, lower alkyl, cycloalkyl, or phenyl;

$R_{18}$ is hydrogen, lower alkyl, lower alkoxy, or phenyl;

n is zero, one, or two;

$R_{19}$ is lower alkyl or

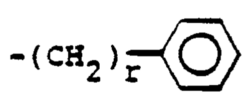 ;

$R_{20}$ is hydrogen, lower alkyl,

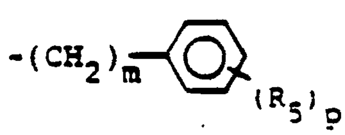 ,

$-(CH_2)_m$-cycloalkyl, 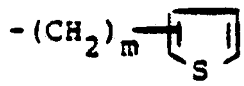 , 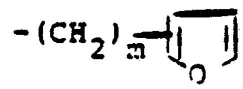 ,

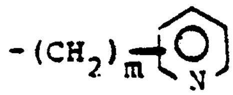 , 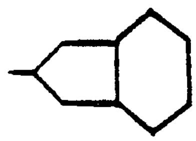 , or 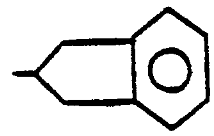 ;

$R_{21}$ is hydrogen, lower alkyl,

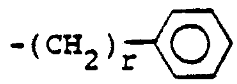 ,

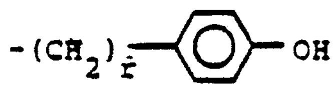 , $-(CH_2)_r-OH$, 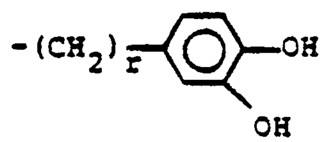 ,

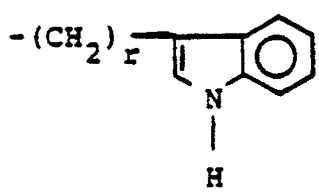 , 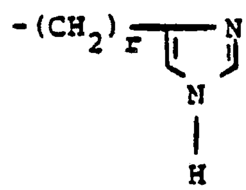 ,

$-(CH_2)_r-NH_2$ , $-(CH_2)_r-SH$ , $-(CH_2)_r$-S-lower alkyl,

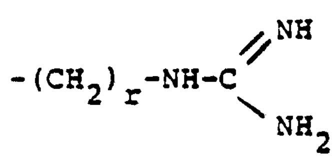 , or 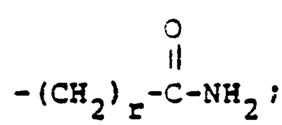 ;

$R_{22}$ is lower alkyl, benzyl, or phenethyl;
$R_{23}$ is hydrogen, lower alkyl, benzyl, or phenethyl; and
$R_6$ is hydrogen, lower alkyl, benzyl, benzhydryl, alkali metal salt ion, alkaline earth metal salt ion,

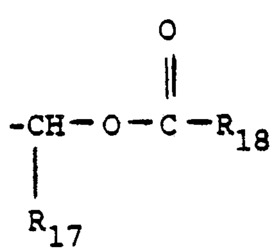 , or $-(CH_2)_2Si(CH_3)_3$ .

**2.** A compound of Claim 1 wherein:
X is

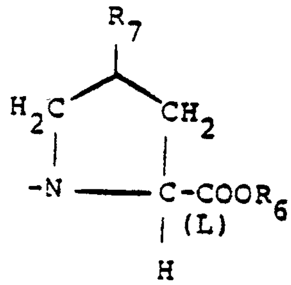
,

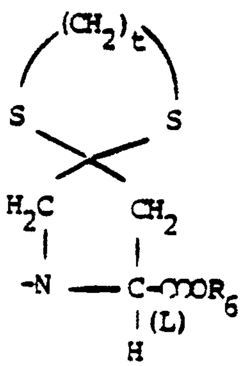
,

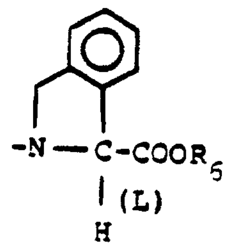
,

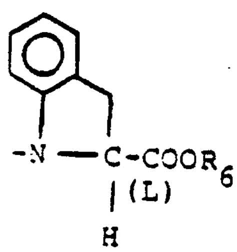
,

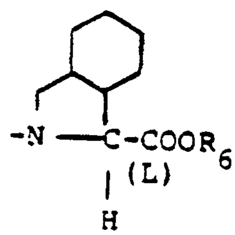
,

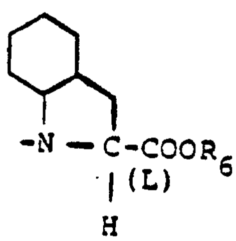
,

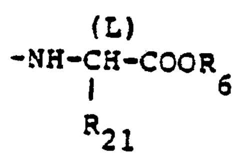
, or

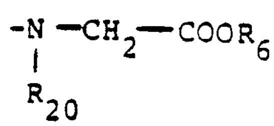
;

$R_7$ is hydrogen, hydroxy, chloro, fluoro, lower alkyl of 1 to 4 carbons, cyclohexyl, amino, -O-lower alkyl wherein lower alkyl is of 1 to 4 carbons, -S-lower alkyl wherein lower alkyl is of 1 to 4 carbons,

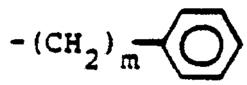 , 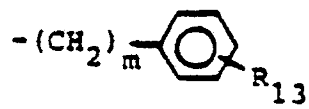 ,

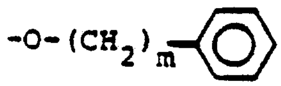 , 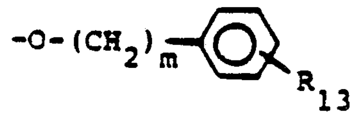 ,

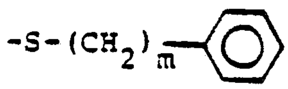 , or 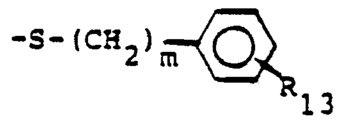 ;

m is zero, one or two;
$R_{13}$ is methyl, methoxy, chloro, fluoro, bromo, methylthio, or hydroxy;
t is 2 or 3;
$R_{20}$ is

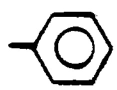 , 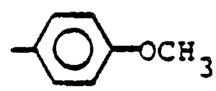 , or

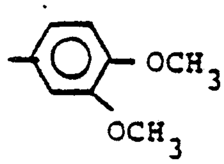 ;

$R_{21}$ is hydrogen, straight or branched chain lower alkyl of 1 to 4 carbons,

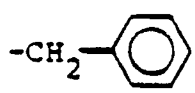 , 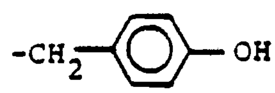 ,

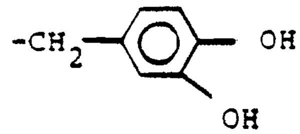 , 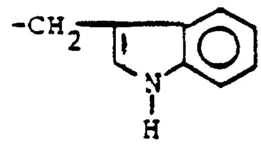 ,

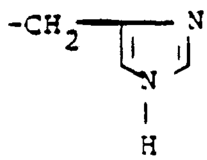 , $-(CH_2)_4-NH_2$ ,

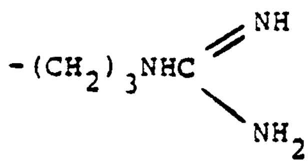 , 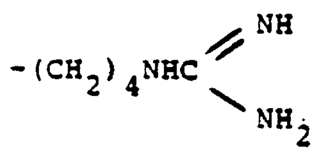 ,

$-CH_2-SH$, $-CH_2-S-CH_3$, 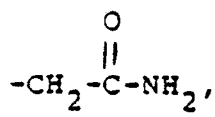 , or 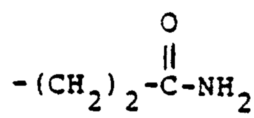 ;

$R_6$ is hydrogen, sodium ion, potassium ion, calcium ion, lithium ion, or

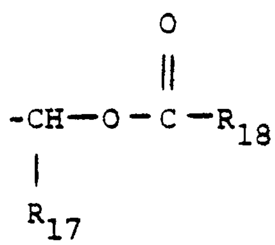 ;

$R_{17}$ is hydrogen, straight or branched chain lower alkyl of 1 to 4 carbons, cyclohexyl, or phenyl; and

$R_{18}$ is hydrogen or straight or branched chain lower alkyl of 1 to 4 carbons.

**3.** A compound of Claim 2 wherein:

$R_1$ is straight or branched chain lower alkyl of 1 to 4 carbons, $-(CH_2)_r-NH_2$, or

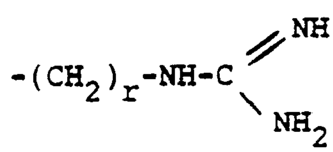 ;

$R_2$ is

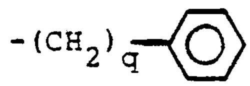 , 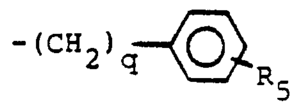 ,

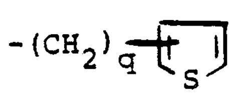 , 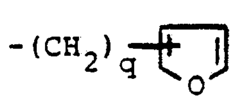 , or 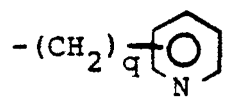 ;

$R_3$ is hydrogen, sodium ion, potassium ion, calcium ion, lithium ion, or

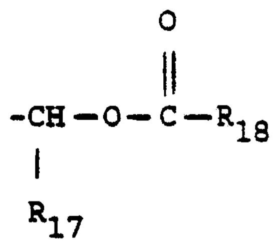 ;

r is an integer from 3 to 5;
q is zero, one, or two;
$R_5$ is methyl, methoxy, methylthio, chloro, bromo, fluoro, or hydroxy;
$R_{17}$ is hydrogen, straight or branched chain lower alkyl of 1 to 4 carbons, cyclohexyl, or phenyl; and
$R_{18}$ is hydrogen or straight or branched chain lower alkyl of 1 to 4 carbons.

**4.** A compound of Claim 3 wherein:
X is

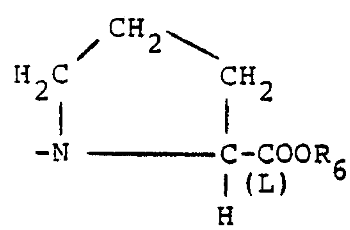 ;

and
$R_6$ is hydrogen, sodium ion, potassium ion, calcium ion, or lithium ion.

**5.** A compound of Claim 4 wherein:
$R_1$ is methyl or $-(CH_2)_4-NH_2$;
$R_2$ is phenyl;
v is 4; and
$R_3$ is hydrogen, sodium ion, potassium ion, calcium ion, or lithium ion.

**6.** The compound of Claim 5 wherein:
$R_1$ is methyl; and
$R_3$ and $R_6$ are both lithium.

**7.** The compound of Claim 6, (S)-1-[2-[[[5-amino-1-(benzoylamino)pentyl]hydroxyphosphinyl]oxy]-1-oxopropyl]-L-proline,dilithium salt.

**8.** The compound of Claim 5 wherein:

$R_1$ is $-(CH_2)_4-NH_2$; and
$R_3$ and $R_6$ are lithium.

**9.** The compound of Claim 8, 1-[(S)-6-amino-2-[[[5-amino-1-(benzoylamino)pentyl]hydroxyphosphinyl]oxy]-1-oxohexyl]-L-proline, dilithium salt.

**10.** A pharmaceutical composition useful for treating hypertension in a mammalian species comprising a pharmaceutically acceptable carrier and an antihypertensively effective amount of a compound of the formula

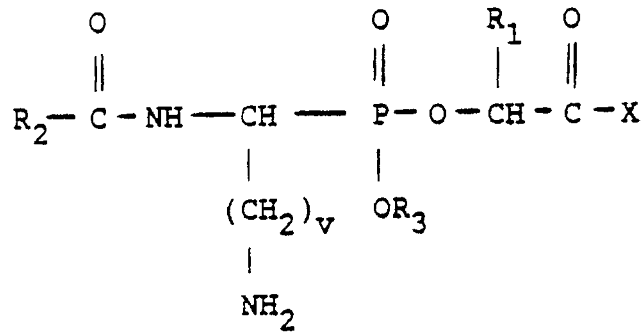

wherein $R_1$, $R_2$, $R_3$, v and X are as defined in Claim 1.

**11.** A pharmaceutical composition useful as an analgesic agent comprising a pharmaceutically acceptable carrier and an analgesically effective amount of an enkephalinase inhibiting compound of the formula

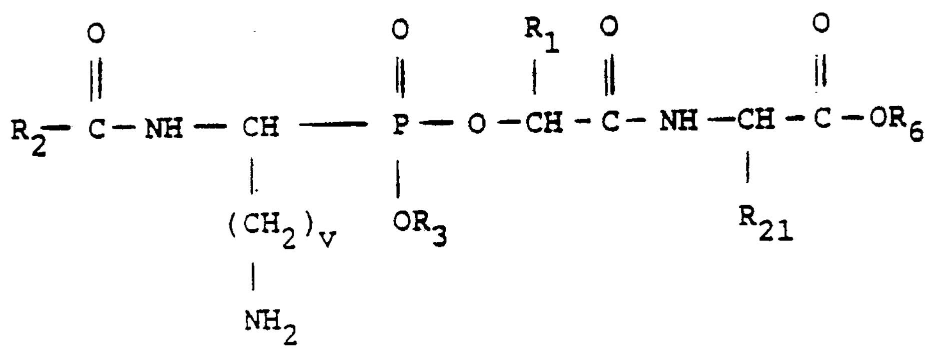

wherein $R_1$, $R_2$, $R_3$, $R_6$, $R_{21}$ and v are as defined in Claim 1.

**12.** A compound of the formula

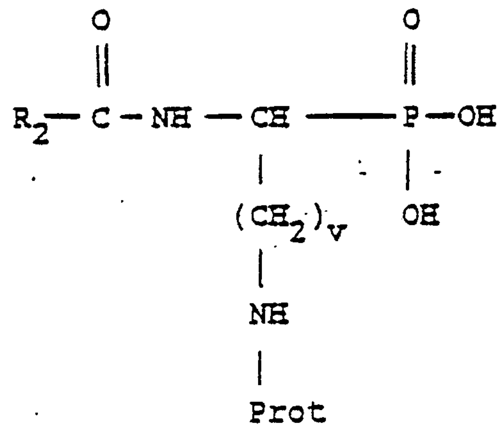

wherein:

$R_2$ is straight or branched chain alkyl of 1 to 10 carbons, $-(CH_2)_q$-cycloalkyl,

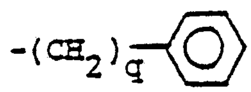,

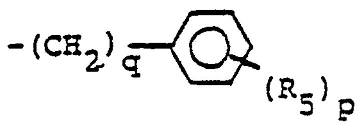, 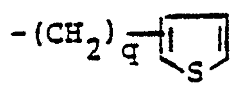,

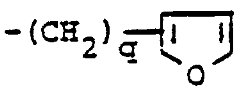, or 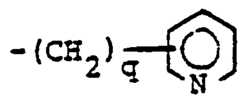;

v is an integer from 3 to 5;

q is zero or an integer from 1 to 7;

$R_5$ is lower alkyl of 1 to 4 carbons, lower alkoxy of 1 to 4 carbons, lower alkylthio of 1 to 4 carbons, chloro, bromo, fluoro, trifluoromethyl or hydroxy;

p is one, two or three provided that p is more than one only if $R_5$ is methyl, methoxy, chloro, bromo, or fluoro; and

Prot is an amino protecting group.

**13.** A compound of Claim 12 wherein:

$R_2$ is

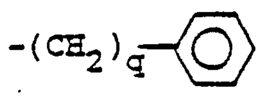, 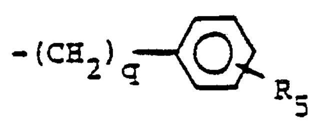,

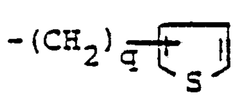, 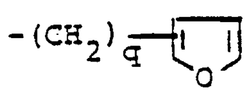, or 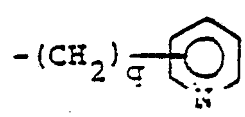;

q is zero, one, or two; and
$R_5$ is methyl, methoxy, methylthio, chloro, bromo, fluoro, or hydroxy.

**14.** The compound of Claim 13 wherein
$R_2$ is

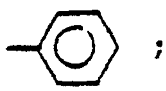

v is four; and
Prot is

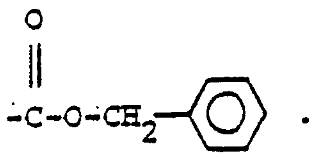

**Revendications**

**1.** Composé de formule

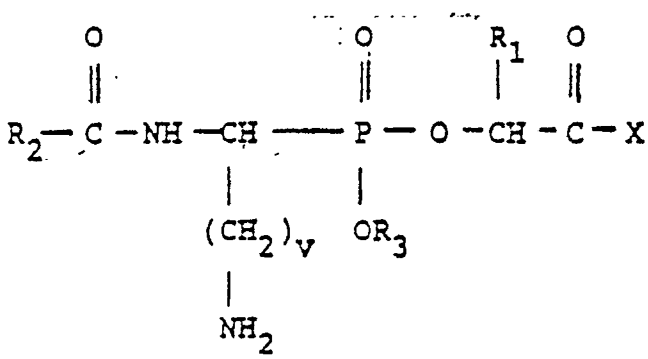

y compris sels pharmaceutiquement acceptables de ce composé, formule dans laquelle :
$R_1$ est l'hydrogène ou un radical alkyle inférieur, $-(CH_2)_r-Cl$,

$-(CH_2)_r-Br$, $-(CH_2)_r-F$, $CF_3$, 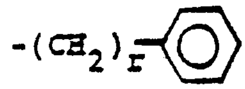 ,

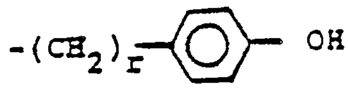 , 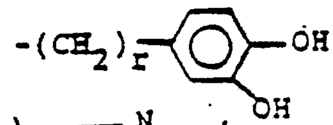 ,

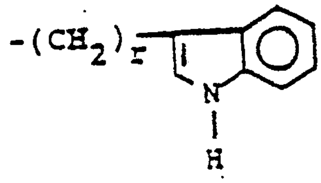 , 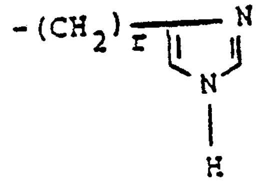 ,

$-(CH_2)_r$-cycloalkyle, $-(CH_2)_r-NH_2$, $-(CH_2)_r-SH$,

$-(CH_2)_r$-S-alkyle inférieur, 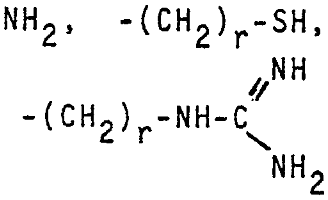 , ou

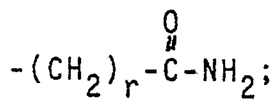 ;

$R_2$ est un radical alkyle à chaîne droite ou ramifiée de 1 à 10 atomes de carbone, $-(CH_2)_q$-cycloalkyle,

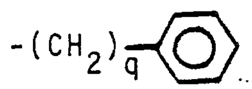 , 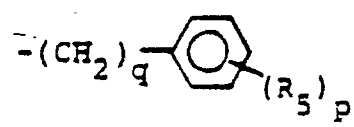 , 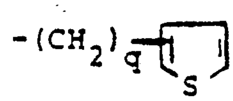 ,

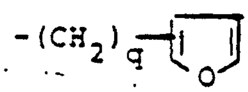 ou 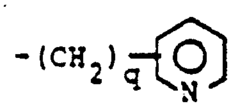 ;

$R_3$ est l'hydrogène, un radical alkyle inférieur ou benzyle, un ion sel de métal alcalin, un ion sel de métal alcalino-terreux, ou

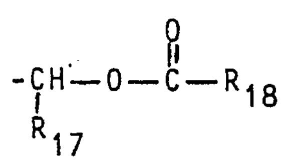 ;

v est un nombre entier de 3 à 5;
r est un nombre entier de 1 à 7;
q est égal à zéro ou est un nombre entier de 1 à 7;
X est un amino- ou imino-acide ou ester de formule

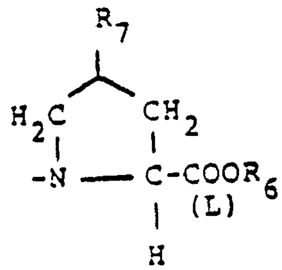 , 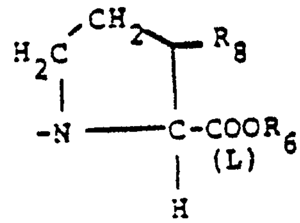 ,

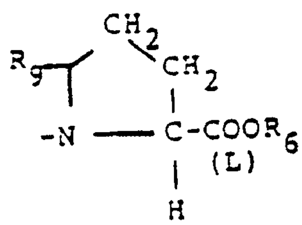 , 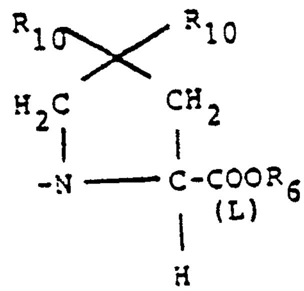 ,

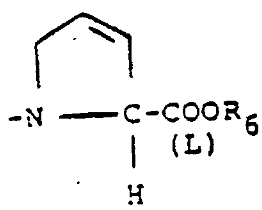 ,

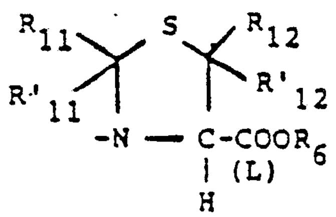 ,

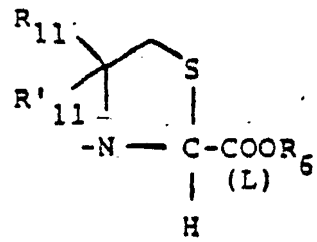 ,

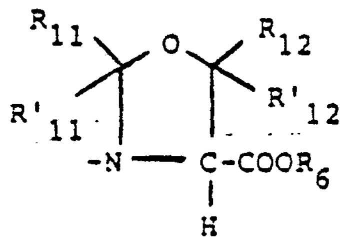

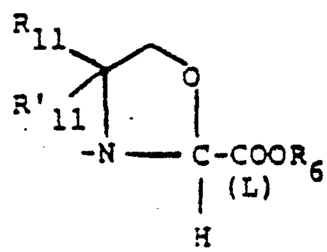

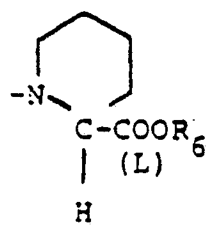 ,

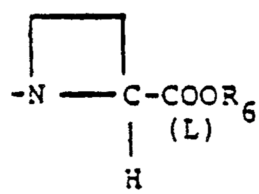 ,

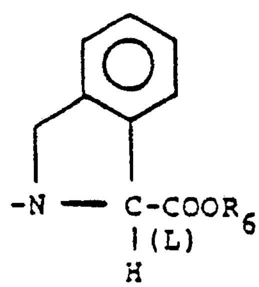 ,

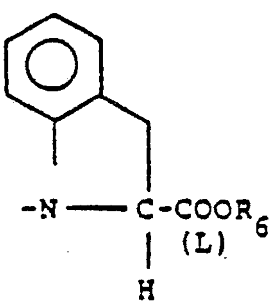 ,

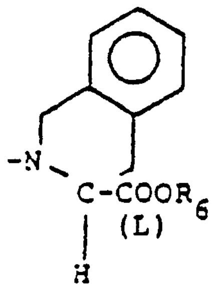 ,

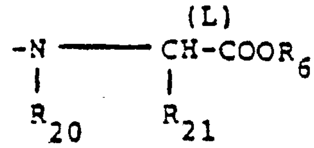

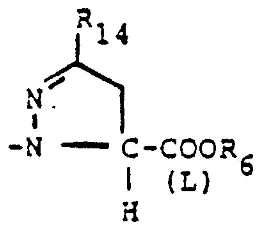 ,

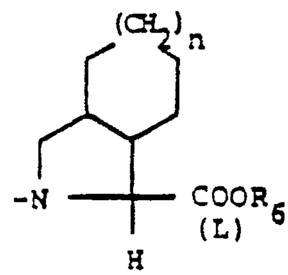 ,

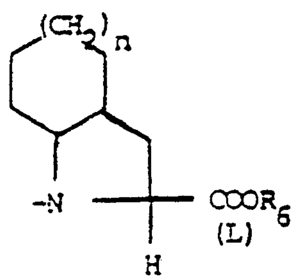 ,

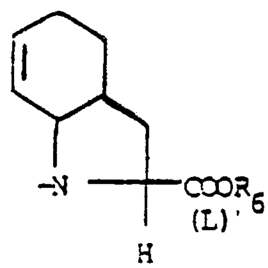 ,

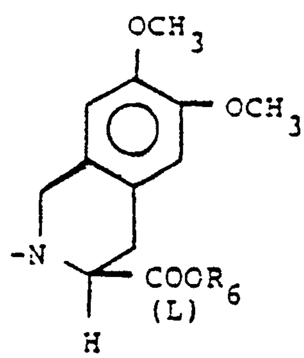 ,

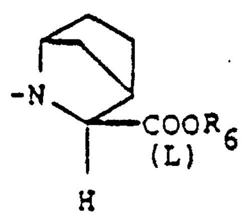 ,

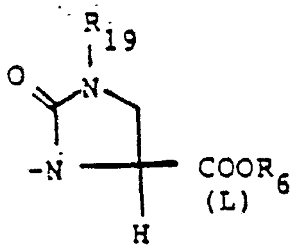 ou 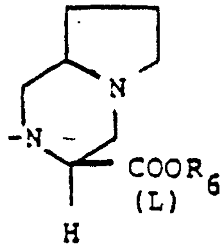 ;

$R_7$ est l'hydrogène, un radical alkyle inférieur, un atome d'halogène, un groupement hydroxy,

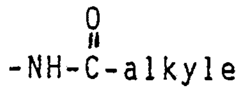 alkyle inférieur, amine, 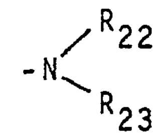 ,

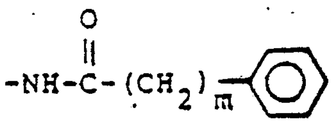 , 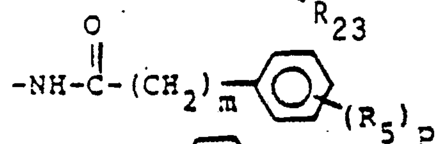 ,

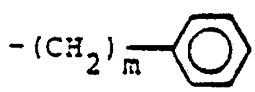 , 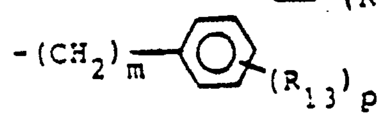 ,

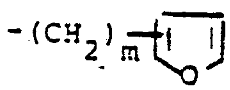 , 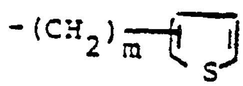 , 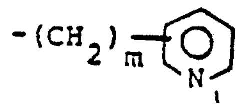 ,

un radical 1- ou 2-naphtyle de formule

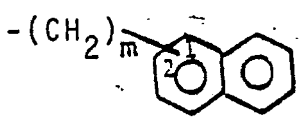 ,

un radical 1- ou 2-naphtyle substitué de formule

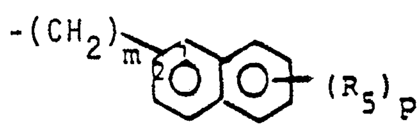 ,

un groupement $-(CH_2)_m$-cycloalkyle,

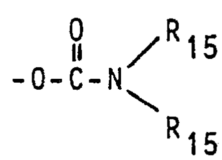 ,

-O-alkyle inférieur,

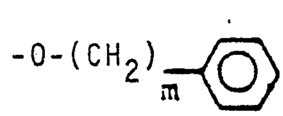 ,

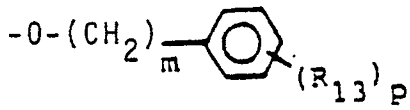 ,

un radical 1- ou 2-naphtyloxy de formule

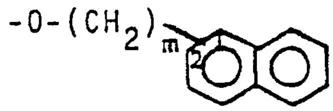 ,

un radical 1- ou 2- naphtyloxy substitué de formule

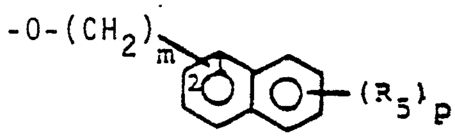 ,

un groupement -S-alkyle inférieur,

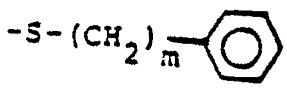 ,

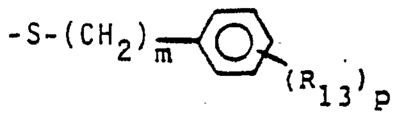 ,

un radical 1- ou 2- naphtylthio de formule

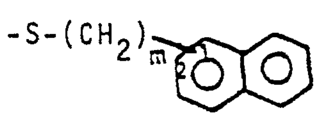 ,

ou un radical 1- ou 2-naphtylthio substitué de formule

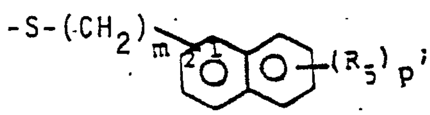 ;

$R_8$ est un radical alkyle inférieur, un atome d'halogène, un groupement

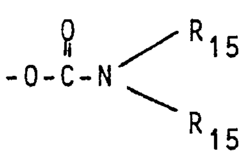 , 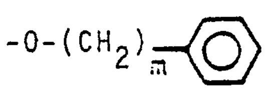 , 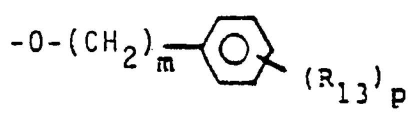 ,

-0- alkyle inférieur, un radical 1- ou 2-naphtyloxy de formule

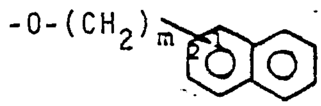 ,

un radical 1- ou 2-naphtyloxy substitué de formule

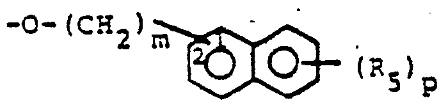 ,

un groupement -S-alkyle inférieur,

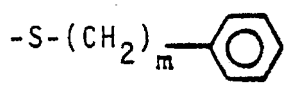 ,

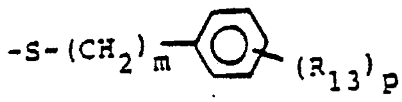 ;

un radical 1- ou 2-naphtylthio de formule

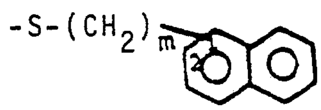

ou un radical 1- ou 2-naphtylthio substitué de formule

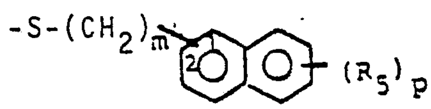 ;

$R_9$ est un radical alkyle inférieur, ou un groupement céto,

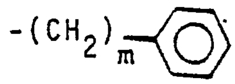 ,

ou

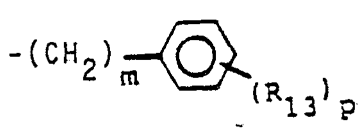 ;

$R_{10}$ est un atome d'halogène ou un groupement Y-$R_{16}$;

$R_{11}$, $R'_{11}$, $R_{12}$ et $R'_{12}$ sont choisis indépendamment entre l'hydrogène et les radicaux alkyle inférieur, ou bien $R'_{11}$, $R_{12}$ et $R'_{12}$ sont l'hydrogène et $R_{11}$ est

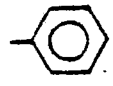 ou 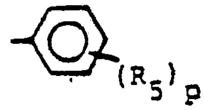 ;

$R_{13}$ est un radical alkyle inférieur de 1 à 4 atomes de carbone, alcoxy inférieur de 1 à 4 atomes de carbone, alkylthio inférieur de 1 à 4 atomes de carbone, un atome de chlore, de brome ou de fluor, ou un groupement trifluorométhyle, hydroxy, phényle, phénoxy, phénylthio, ou phénylméthyle;

$R_5$ est un radical alkyle inférieur de 1 à 4 atomes de carbone, alcoxy inférieur de 1 à 4 atomes de carbone, alkylthio inférieur de 1 à 4 atomes de carbone, un atome de chlore, de brome ou de fluor, ou un groupement trifluorométhyle ou hydroxy;

m est égal à zéro, un, deux, trois ou quatre;

p est égal à un, deux ou trois, à condition que p ne soit plus grand que un que si $R_{13}$ ou $R_5$ est un radical méthyle ou méthoxy, ou un atome de chlore, de brome ou de fluor;

$R_{14}$ est l'hydrogène, un radical alkyle inférieur, ou un groupement de formule

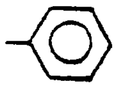 , 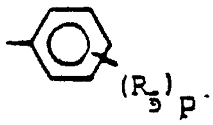 , 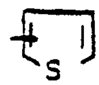 , 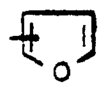 , ou 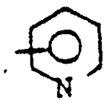 ;

$R_{15}$ est l'hydrogène ou un radical alkyle inférieur de 1 à 4 atomes de carbone;

Y est l'oxygène ou le soufre;

$R_{16}$ est un radical alkyle inférieur de 1 à 4 atomes de carbone, un groupement de formule

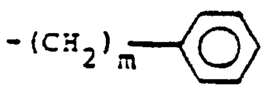 ou 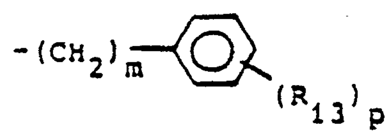 ,

ou bien les groupements $R_{16}$ forment ensemble un cycle à 5 ou 6 chaînons non substitué, ou bien ce même cycle dans lequel un ou plusieurs des atomes de carbone portent un substituant alkyle inférieur de 1 à 4 atomes de carbone ou un substituant di(alkyle inférieur de 1 à 4 atomes de carbone);

$R_{17}$ est l'hydrogène ou un radical alkyle inférieur, cycloalkyle, ou phényle;

$R_{18}$ est l'hydrogène ou un radical alkyle inférieur, alcoxy inférieur ou phényle;

n est égal à zéro, un ou deux;

$R_{19}$ est un radical alkyle inférieur ou

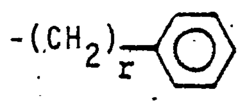 ;

$R_{20}$ est l'hydrogène, un radical alkyle inférieur, ou un groupement de formule

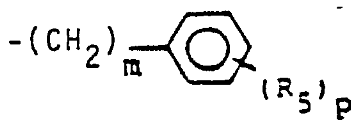 ,

$-(CH_2)_m$-cycloalkyle,

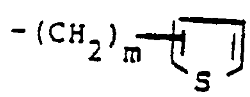 , 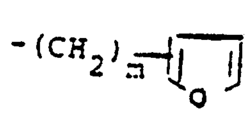 ,

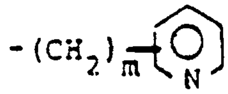 , 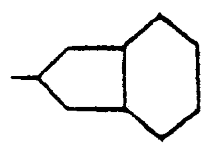 , ou 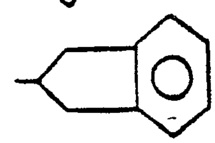 ;

$R_{21}$ est l'hydrogène, un radical alkyle inférieur, ou un groupement de formule

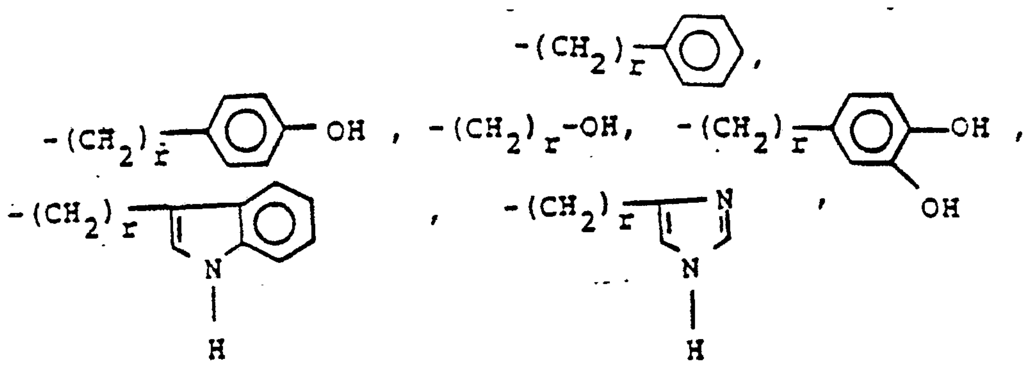

$-(CH_2)_r-OH$,

$-(CH_2)_r-NH_2$, $-(CH_2)_r-SH$, $-(CH_2)_r-S\text{-alkyle inférieur}$,

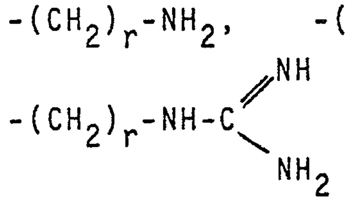

ou $-(CH_2)_r-\overset{O}{\overset{\|}{C}}-NH_2$ ;

$R_{22}$ est un radical alkyle inférieur, benzyle ou phénéthyle;

$R_{23}$ est l'hydrogène ou un radical alkyle inférieur, benzyle ou phénéthyle; et

$R_6$ est l'hydrogène, un radical alkyle inférieur, benzyle ou benzhydryle, un ion sel de métal alcalin, un ion sel de métal alcalino-terreux, ou un groupement de formule

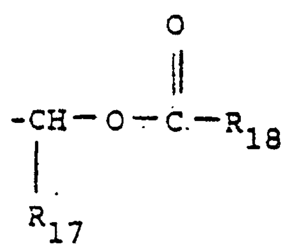

, ou $-(CH_2)_2Si(CH_3)_3$.

2. Composé selon la revendication 1, dans lequel X est

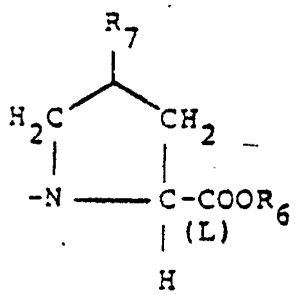 ,

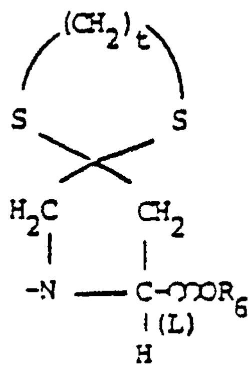 ,

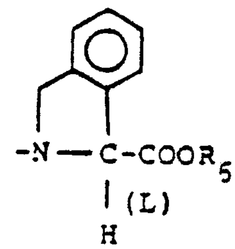 ,

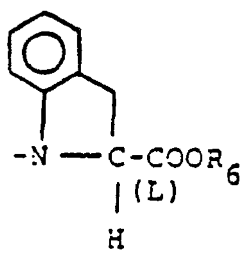 ,

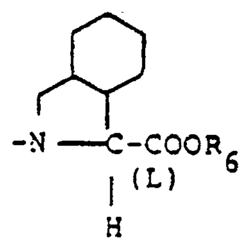 ,

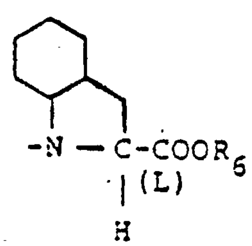 ,

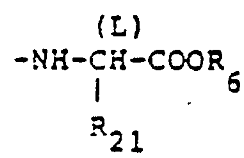 , ou

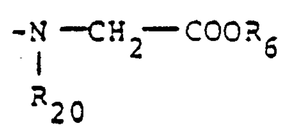 ;

$R_7$ est l'hydrogène, un groupement hydroxy, un atome de chlore ou de fluor, un radical alkyle inférieur de 1 à 4 atomes de carbone, un groupement cyclohexyle, amine, -O-alkyle inférieur dans lequel le radical alkyle inférieur a de 1 à 4 atomes de carbone, -S-alkyle inférieur dans lequel le radical alkyle inférieur a de 1 à 4 atome de carbone, ou un groupement de formule

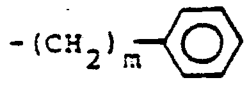 , 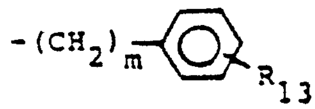 ,

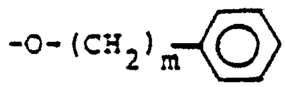 , 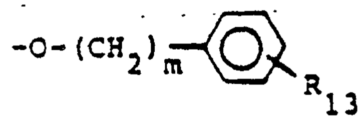 ,

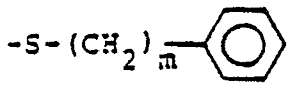 , ou 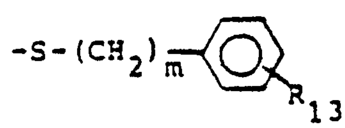 ;

m est égal à zéro, un ou deux;

$R_{13}$ est le radical méthyle ou méthoxy, un atome de chlore, de fluor ou de brome, ou le groupement méthylthio ou hydroxy;

t est égal à 2 ou 3;

$R_{20}$ est

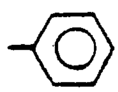 , 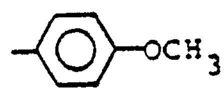 ou 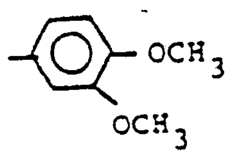 ;

$R_{21}$ est l'hydrogène, un radical alkyle inférieur à chaîne droite ou ramifiée de 1 à 4 atomes de carbone, ou un groupement de formule

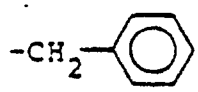 , 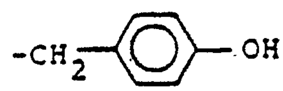 ,

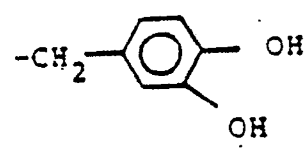 , 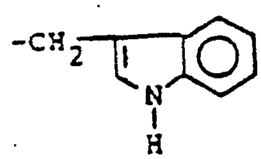 ,

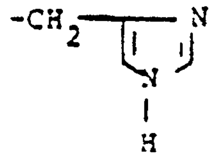 , $-(CH_2)_4-NH_2$ ,

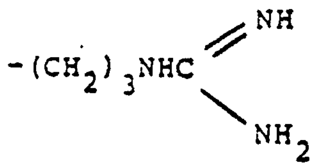 , 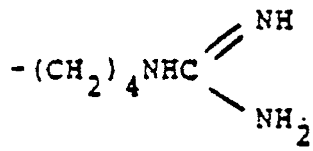 ,

$-CH_2-SH$, $-CH_2-S-CH_3$, 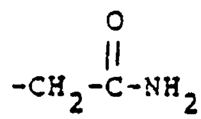, ou 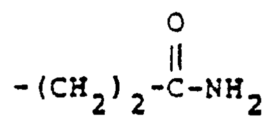 ;

$R_6$ est l'hydrogène, l'ion sodium, l'ion potassium, l'ion calcium, l'ion lithium, ou

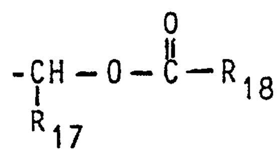 ;

$R_{17}$ est l'hydrogène, un radical alkyle inférieur à chaîne droite ou ramifiée de 1 à 4 atomes de carbone, ou un radical cyclohexyle ou phényle; et

$R_{18}$ est l'hydrogène ou un radical alkyle inférieur à chaîne droite ou ramifiée de 1 à 4 atomes de carbone.

**3.** Composé selon la revendication 2, dans lequel :

$R_1$ est un radical alkyle inférieur à chaîne droite ou ramifiée de 1 à 4 atomes de carbone, ou un groupement de formule $-(CH_2)_r-NH_2$, ou

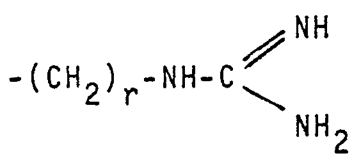 ;

$R_2$ est

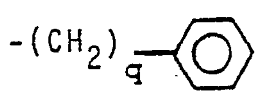 , 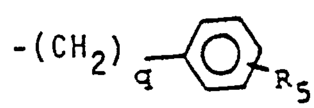 ,

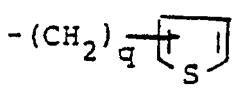 , 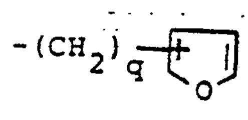 , ou 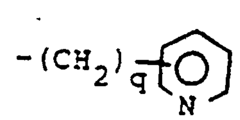 ;

$R_3$ est l'hydrogène, l'ion sodium, l'ion potassium, l'ion calcium, l'ion lithium, ou

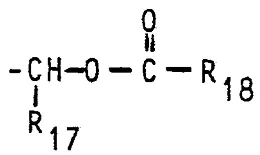

r est un nombre entier de 3 à 5;

q est égal à zéro, un ou deux;

$R_5$ est un radical méthyle, méthoxy, méthylthio, un atome de chlore, de brome ou de fluor, ou le groupement hydroxy;

$R_{17}$ est l'hydrogène, un radical alkyle inférieur à chaîne droite ou ramifiée de 1 à 4 atomes de carbone, ou un radical cyclohexyle ou phényle; et

$R_{18}$ est l'hydrogène ou un radical alkyle inférieur à chaîne droite ou ramifiée de 1 à 4 atomes de carbone.

**4.** Composé selon la revendication 3, dans lequel :

X est

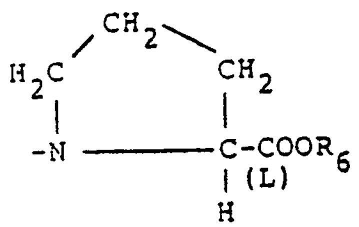

;

et

$R_6$ est l'hydrogène, l'ion sodium, l'ion potassium, l'ion calcium ou l'ion lithium.

**5.** Composé selon la revendication 4, dans lequel :

$R_1$ est le radical méthyle ou $-(CH_2)_4-NH_2$;

$R_2$ est le radical phényle;

Y est égal à 4; et

$R_3$ est l'hydrogène, l'ion sodium, l'ion potassium, l'ion calcium, ou l'ion lithium.

**6.** Composé selon la revendication 5, dans lequel :

$R_1$ est le radical méthyle; et

$R_3$ et $R_6$ sont tous deux le lithium.

**7.** Composé selon la revendication 6, qui est le sel de dilithium de la (S)-1-[2-[[[5-amino-1-(benzoylamino)-pentyl]hydroxyphosphinyl]oxy]-1-oxopropyl]-L-proline.

**8.** Composé selon la revendication 5, dans lequel:

$R_1$ est $-(CH_2)_4-NH_2$; et

$R_3$ et $R_6$ sont le lithium.

**9.** Composé selon la revendication 8, qui est le sel de dilithium de la 1-[(S)-6-amino-2-[[[5-amino-1-(benzoylamino)pentyl]hydroxyphosphinyl]oxy]-1-oxohexyl]-L-proline.

**10.** Composition pharmaceutique utilisable pour traiter l'hypertension chez une espèce mammifère, comprenant un porteur pharmaceutiquement acceptable et une quantité efficace, du point de vue anti-hypertenseur, d'un composé de formule

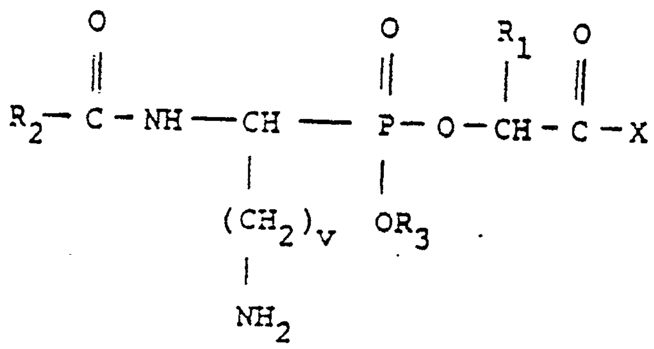

dans làquelle $R_1$, $R_2$, $R_3$, v et X sont tels que définis dans la revendication 1.

**11.** Composition pharmaceutique utilisable comme analgésique, comprenant un porteur pharmaceutiquement acceptable et une quantité efficace, du point de vue analgésique, d'un composé inhibiteur de l'enképhalinase, de formule

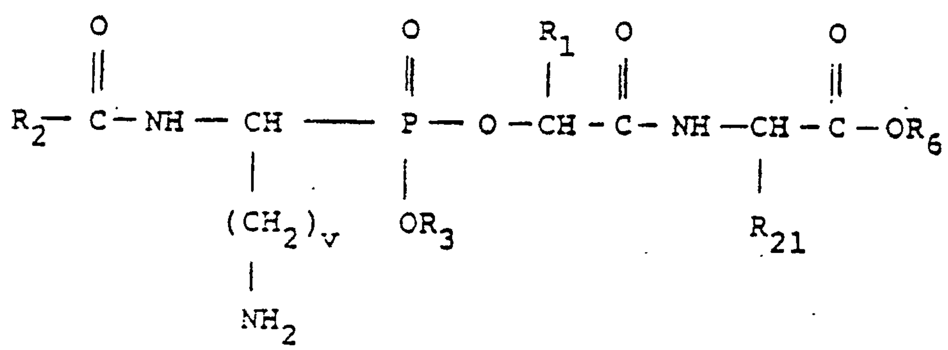

dans laquelle $R_1$,$R_2$, $R_3$,$R_6$,$R_{21}$ et v sont tels que définis dans la revendication 1.

**12.** Composé de formule

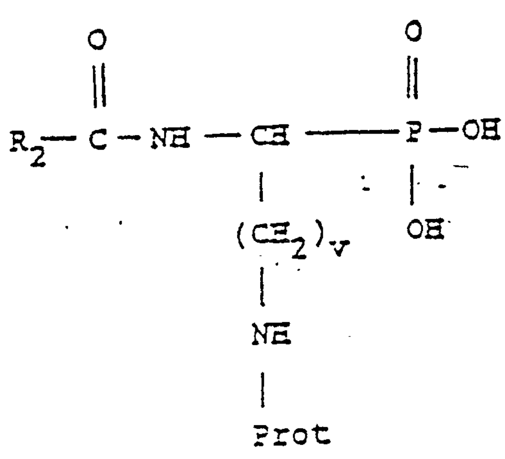

dans laquelle :

$R_2$ est un radical alkyle à chaîne droite ou ramifiée de 1 à 10 atomes de carbone, ou un groupement -$(CH_2)$q-cycloalkyle,

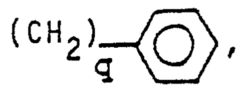,

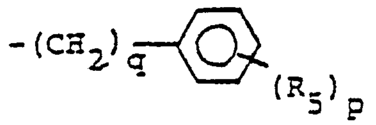, 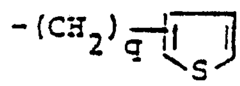,

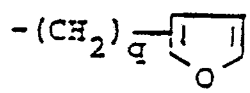, ou 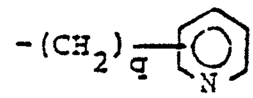;

v est un nombre entier de 3 à 5;

q est égal à zéro ou est un nombre entier de 1 à 7;

$R_5$ est un radical alkyle inférieur de 1 à 4 atomes de carbone, alcoxy inférieur de 1 à 4 atomes de carbone, alkylthio inférieur de 1 à 4 atomes de carbone, un atome de chlore, de brome ou de fluor, ou un groupement trifluorométhyle ou hydroxy;

p est égal à un, deux ou trois, à la condition que p ne soit plus grand que un que si $R_5$ est un radical méthyle ou méthoxy ou un atome de chlore, de brome ou de fluor; et

Prot est un groupement protecteur de la fonction amine.

**13.** Composé selon la revendication 12, dans lequel :

$R_2$ est

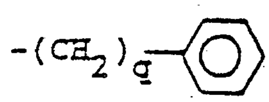, 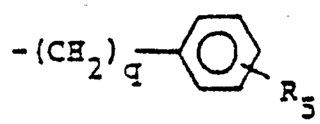,

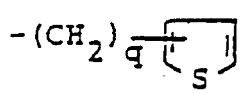, 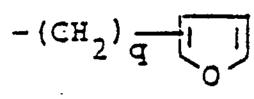, ou 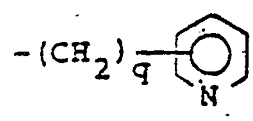;

q est égal à zéro, un ou deux; et

$R_5$ est un radical méthyle, méthoxy ou méthylthio, un atome de chlore, de brome ou de fluor, ou le groupement hydroxy.

**14.** Composé selon la revendication 13, dans lequel

$R_2$ est

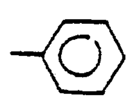;

v est égal à quatre; et

Prot est

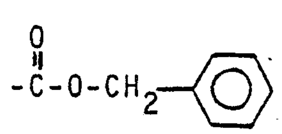.

**Patentansprüche**

1. Verbindung der Formel

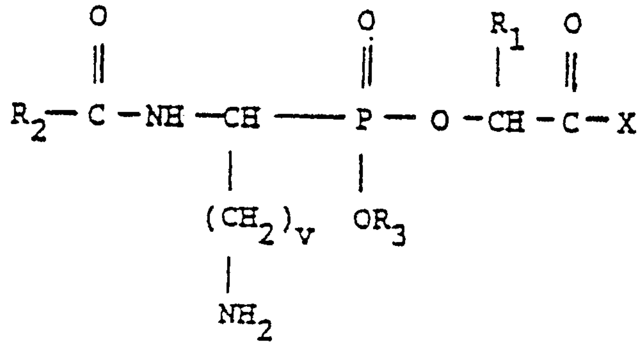

,

einschließlich ein pharmazeutisch verträgliches Salz davon, wobei $R_1$ ein Wasserstoffatom, ein Niederalkylrest, einer der Reste

$-(CH_2)_r-Cl$, $-(CH_2)_r-Br$, $-(CH_2)_r-F$, $-CF_3$, 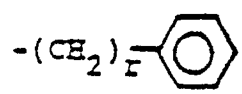 ,

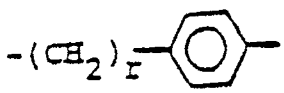 , 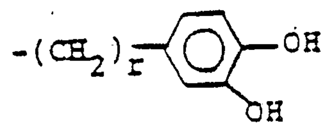 ,

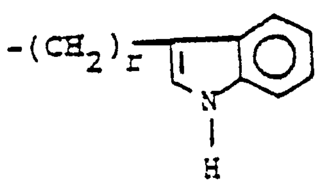 , 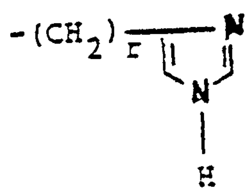

$-(CH_2)_r$-Cycloalkyl, $-(CH_2)_r-NH_2$, $-(CH_2)_r-SH$,

$-(CH_2)_r$-S-Niederalkyl, 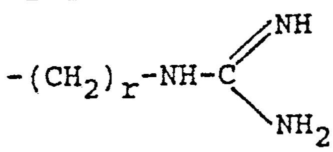 , oder

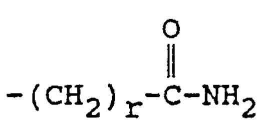 ist;

$R_2$ ein gerad- oder verzweigtkettiger Alkylrest mit 1 bis 10 Kohlenstoffatomen, einer der Reste

$-(CH_2)_q$-Cycloalkyl, 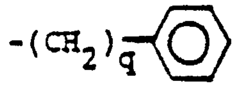 ,

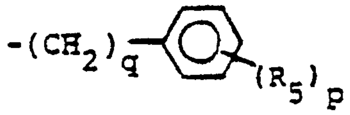 , 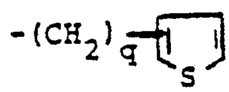 ,

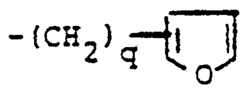 oder 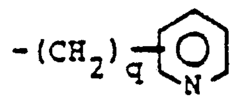 ist;

$R_3$ ein Wasserstoffatom, ein Niederalkylrest, eine Benzylgruppe, ein Alkalimetallsalzion, ein Erdalkalimetallsalzion oder der Rest

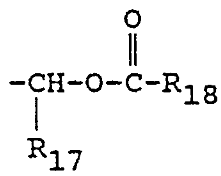 ist;

v eine ganze Zahl von 3 bis 5 ist;
r eine ganze Zahl von 1 bis 7 ist;
q 0 oder eine ganze Zahl von 1 bis 7 ist;
X eine Amino- oder Iminosäure oder ein Ester der Formel

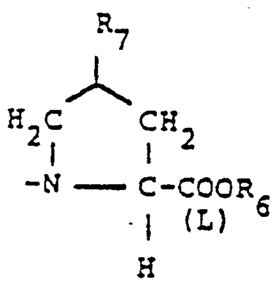 , 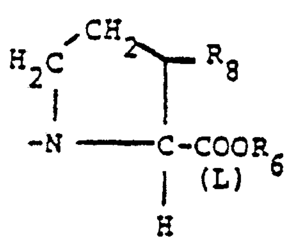 ,

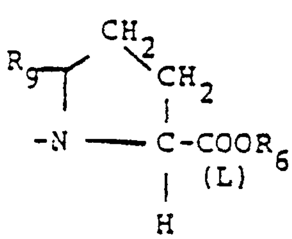 , 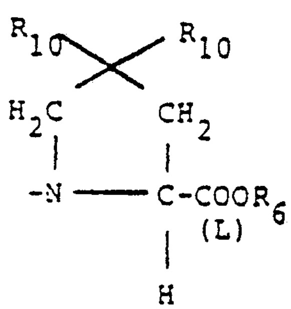 , 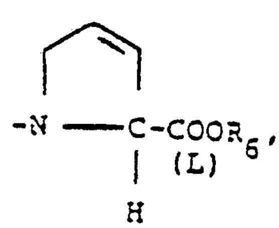

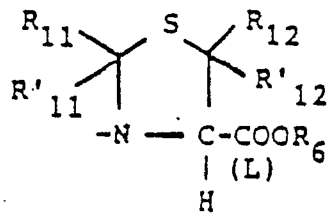 , 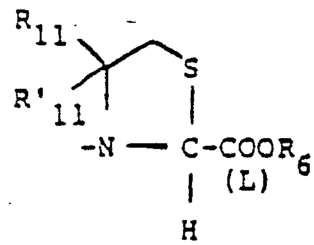 ,

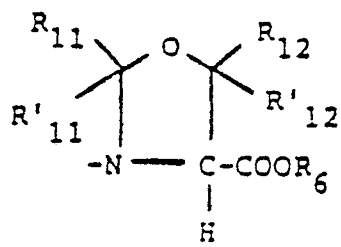

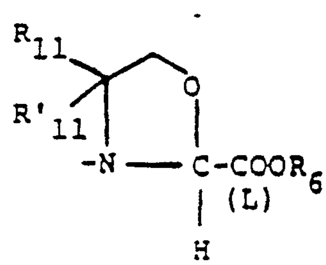

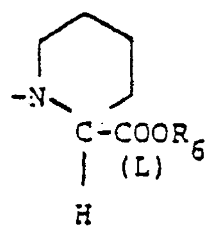 , 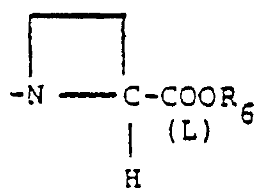 ,

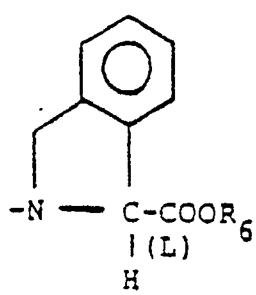 , 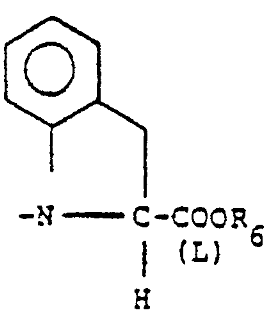 ,

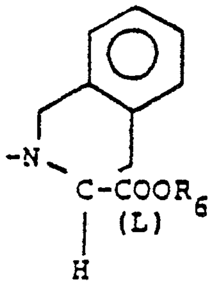

,

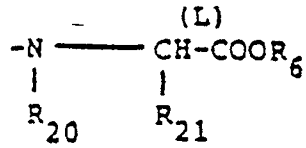

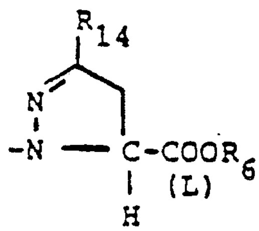

,

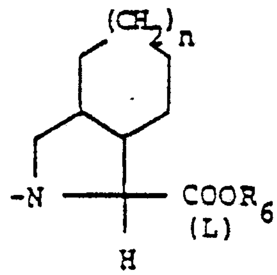

,

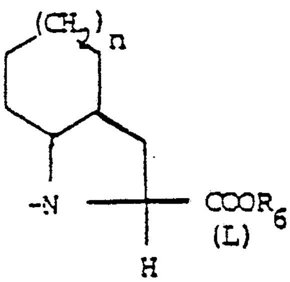

,

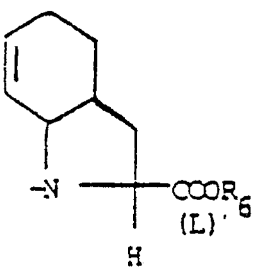

,

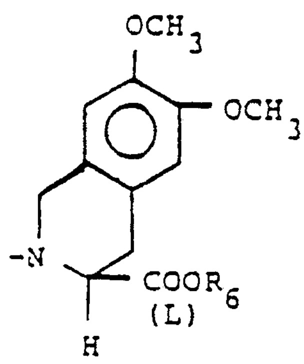

,

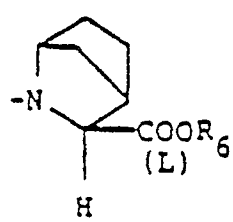

,

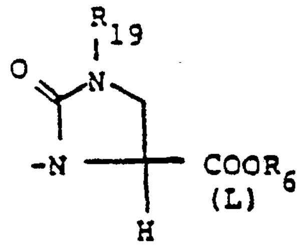 oder 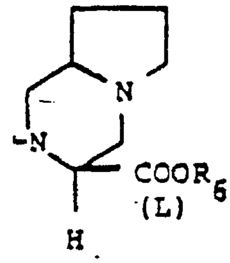 ; ist;

$R_7$ ein Wasserstoffatom, ein Niederalkylrest, ein Halogenatom, eine Hydroxylgruppe, ein

$-NH-\overset{O}{\overset{\|}{C}}-$Niederalkylrest, eine Aminogruppe,

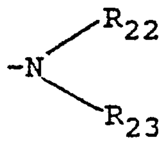 ,

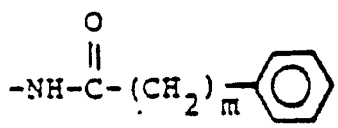 , 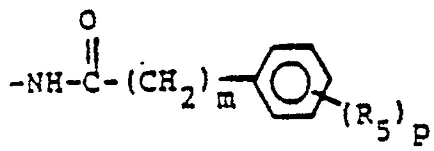 ,

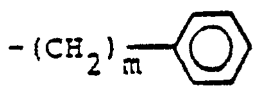 , 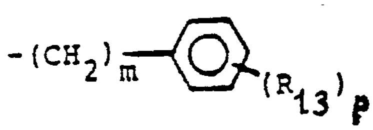 ,

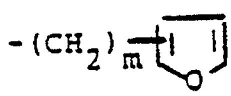 , 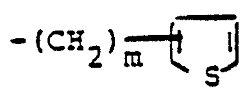 , 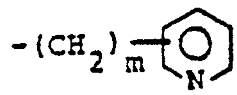 ,

ein 1- oder 2-Naphthylrest der Formel

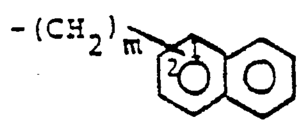 ,

ein substituierter 1- oder 2-Naphthylrest der Formel

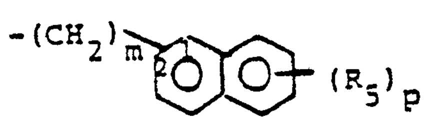 ,

ein $-(CH_2)_m$-Cycloalkylrest, der Rest

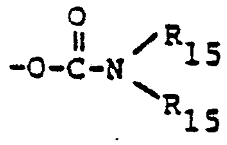,

ein -O-Niederalkylrest, einer der Reste

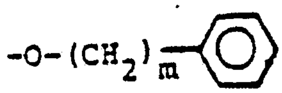, 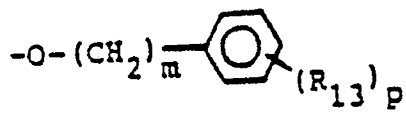,

ein 1- oder 2-Naphthyloxyrest der Formel

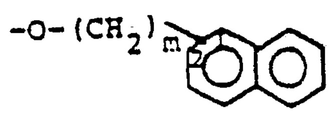

ein substituierter 1- oder 2-Naphthyloxyrest der Formel

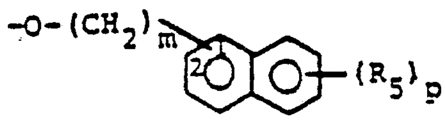,

ein -S-Niederalkylrest, einer der Reste

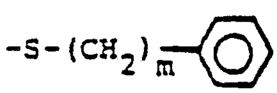, 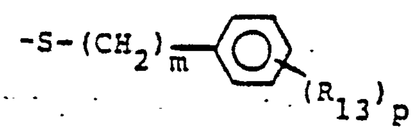,

ein 1- oder 2-Naphthylthiorest der Formel

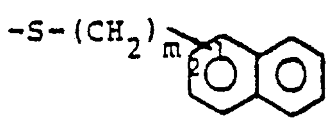

oder ein substituierter 1- oder 2-Naphthylthiorest der Formel

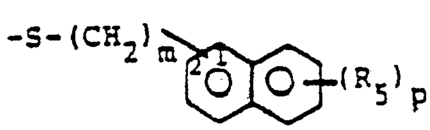;

ist;

$R_8$ ist ein Niederalkylrest, ein Halogenatom, einer der Reste

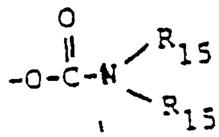 ,

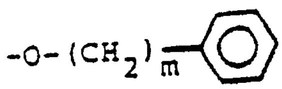 , 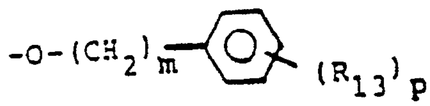 ,

ein -O-Niederalkylrest, ein 1- oder 2-Naphthyloxyrest der Formel

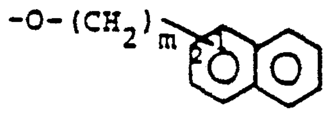 ,

ein substituierter 1- oder 2-Naphthyloxyrest der Formel

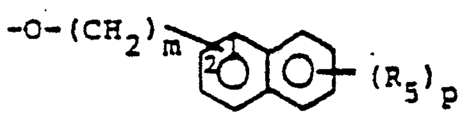 ,

ein -S-Niederalkylrest, einer der Reste

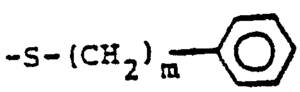 , 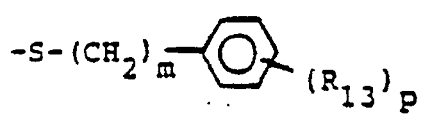 ,

ein 1- oder 2-Naphthylthiorest der Formel

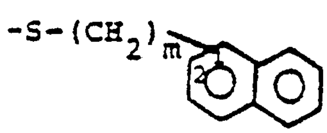 ,

oder ein substituierter 1- oder 2-Naphthylthiorest der Formel

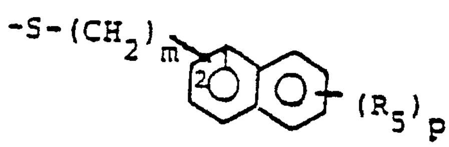

ist;

$R_9$ ein Niederalkylrest, eine Ketogruppe, der Rest

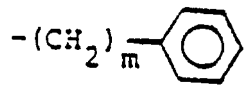 oder der Rest 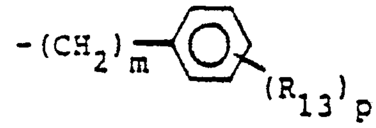 ist;

$R_{10}$ ein Halogenatom oder der Rest Y-$R_{16}$ ist;
$R_{11}$, $R'_{11}$, $R_{12}$ und $R'_{12}$ unabhängig voneinander ausgewählt werden aus Wasserstoffatomen und Niederalkylresten oder $R'_{11}$, $R_{12}$ und $R'_{12}$ Wasserstoffatome sind und $R_{11}$ die Gruppe

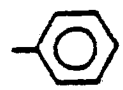 oder der Rest 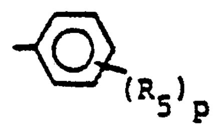 ist;

$R_{13}$ ein Niederalkylrest mit 1 bis 4 Kohlenstoffatomen, ein Niederalkoxyrest mit 1 bis 4 Kohlenstoffatomen, ein Niederalkylthiorest mit 1 bis 4 Kohlenstoffatomen, ein Chlor-, Brom- oder Fluoratom, eine der Gruppen Trifluormethyl, Hydroxyl, Phenyl, Phenoxy-, Phenylthio oder Phenylmethyl ist;
$R_5$ ein Niederalkylrest mit 1 bis 4 Kohlenstoffatomen, ein Niederalkoxyrest mit 1 bis 4 Kohlenstoffatomen, ein Niederalkylthiorest mit 1 bis 4 Kohlenstoffatomen, ein Chlor-, Brom- oder Fluoratom, eine Trifluormethyl- oder Hydroxylgruppe ist;
m den Wert 0, 1, 2, 3 oder 4 aufweist;
p den Wert 1, 2 oder 3 aufweist, mit der Maßgabe, daß p nur dann größer als 1 ist, wenn $R_{13}$ oder $R_5$ eine Methyloder Methoxygruppe, ein Chlor-, Brom- oder Fluoratom ist;
$R_{14}$ ein Wasserstoffatom, ein Niederalkylrest, die Gruppe

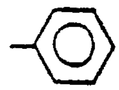 ,

einer der Reste

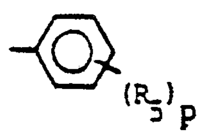 , 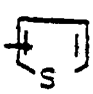 , 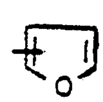 , oder 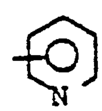

ist;

$R_{15}$ ein Wasserstoffatom oder ein Niederalkylrest mit 1 bis 4 Kohlenstoffatomen ist;
Y ein Sauerstoff- oder Schwefelatom ist;
$R_{16}$ ein Niederalkylrest mit 1 bis 4 Kohlenstoffatomen, einer der Reste

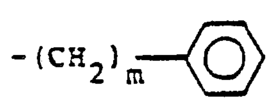 , 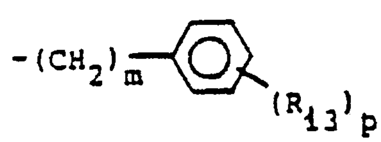 ,

oder die $R_{16}$-Gruppen zusammen einen nicht-substituierten 5- oder 6-gliedrigen Ring aufbauen oder einen solchen Ring, bei dem eines oder mehrere der Kohlenstoffatome mit einem Niederalkylrest mit 1 bis 4 Kohlenstoffatomen oder einem Di(niederalkylrest mit 1 bis 4 Kohlenstoffatomen) substituiert ist bzw. sind;

$R_{17}$ ein Wasserstoffatom, ein Niederalkyl- oder Cycloalkylrest, oder eine Phenylgruppe ist;
$R_{18}$ ein Wasserstoffatom, ein Niederalkyl- oder Niederalkoxyrest oder eine Phenylgruppe ist;
n den Wert 0, 1 oder 2 aufweist;
$R_{19}$ ein Niederalkylrest oder der Rest

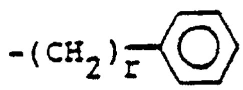

ist;

$R_{20}$ ein Wasserstoffatom, ein Niederalkylrest, einer der Reste

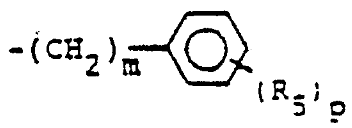 , $-(CH_2)_m$-Cycloalkyl,

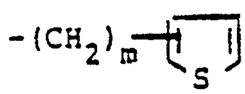 , 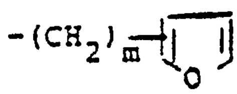 , 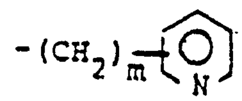 ,

eine der Gruppen 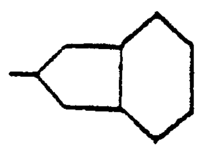 oder 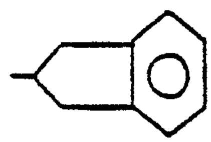 ist;

$R_{21}$ ein Wasserstoffatom, ein Niederalkylrest, einer der Reste

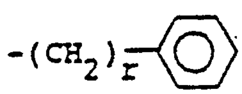 ,

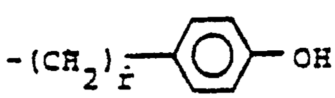 , $-(CH_2)_r$-OH, 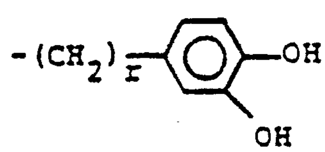 ,

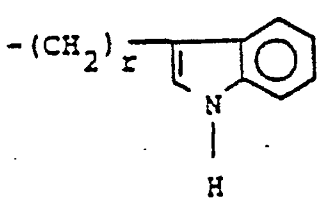 , 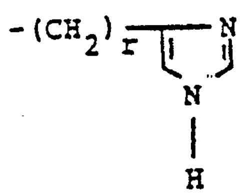 ,

$-(CH_2)_r-NH_2$, $-(CH_2)_r$-SH, $-(CH_2)_r$-S-Niederalkyl,

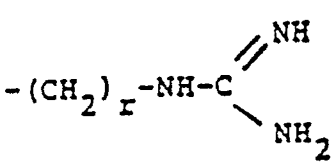 oder 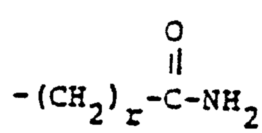

ist;

$R_{22}$ ein Niederalkylrest, eine Benzyl- oder Phenethylgruppe ist;
$R_{23}$ ein Wasserstoffatom, ein Niederalkylrest, eine Benzyl- oder Phenethylgruppe ist; und
$R_6$ ein Wasserstoffatom, ein Niederalkylrest, eine Benzyl- oder Benzhydrylgruppe, ein Alkalimetallsalzion, ein Erdalkalimetallsalzion, einer der Reste

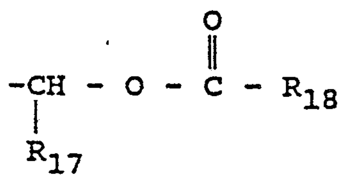

oder $-(CH_2)_2Si(CH_3)_3$ ist.

2. Verbindung der Formel 1, wobei X einer der Reste

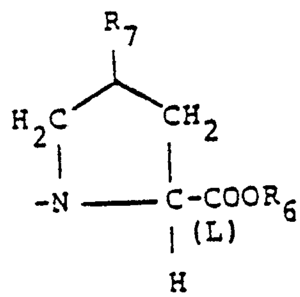

,

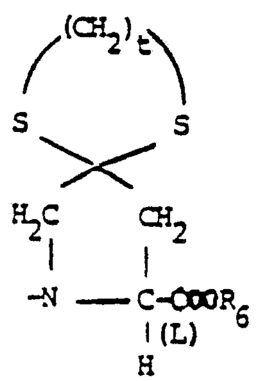

,

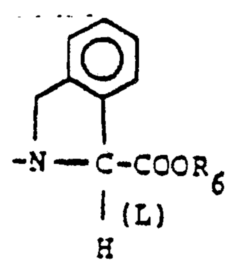

,

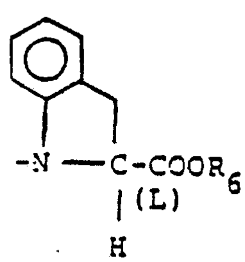

,

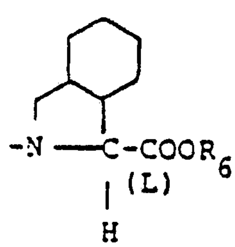

,

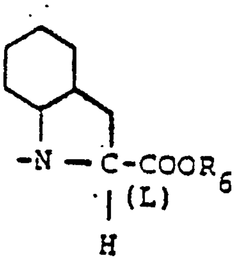 , 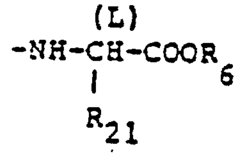 oder

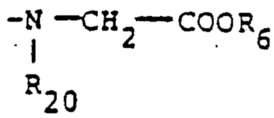 ist;

$R_7$ ein Wasserstoffatom, eine Hydroxylgruppe, ein Chlor-, oder Fluoratom, ein Niederalkylrest mit 1 bis 4 Kohlenstoffatomen, eine Cyclohexyl- oder Aminogruppe, ein -O-Niederalkylrest, wobei der Niederalkylrest 1 bis 4 Kohlenstoffatome aufweist, ein -S-Niederalkylrest, wobei der Niederalkylrest 1 bis 4 Kohlenstoffe aufweist, einer der Reste

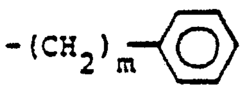 , 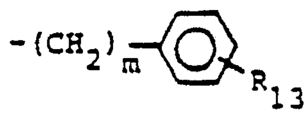 ,

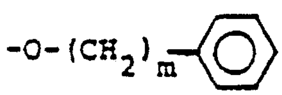 , 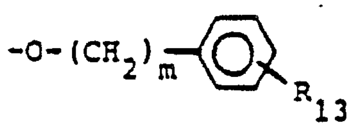 ,

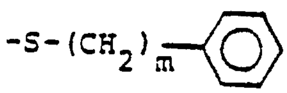 oder 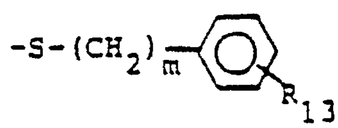

ist;
m den Wert 0, 1 oder 2 aufweist;
$R_{13}$ eine Methyl- oder Methoxygruppe, ein Chlor-, Fluor- oder Bromatom, eine Methylthio- oder Hydroxylgruppe ist,
t den Wert 2 oder 3 aufweist,
$R_{20}$ eine der Gruppen

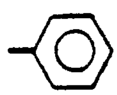 , 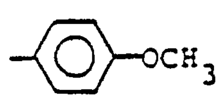 oder 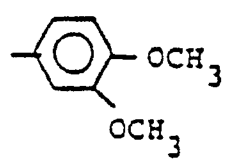 ist;

$R_{21}$ ein Wasserstoffatom, ein gerad- oder verzweigtkettiger Niederalkylrest mit 1 bis 4 Kohlenstoffatomen, eine der Gruppen

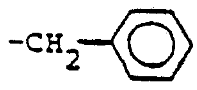 , 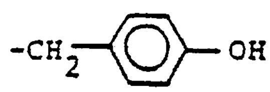 ,

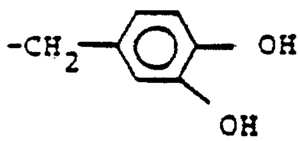 , 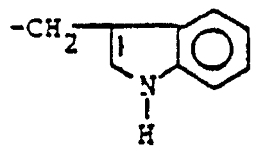 ,

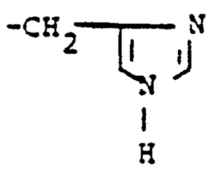 , $-(CH_2)_4-NH_2$ ,

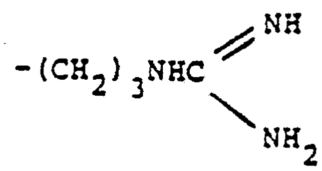 , 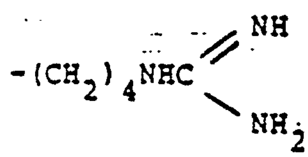 ,

$-CH_2-SH$, $-CH_2-S-CH_3$, $-CH_2-\overset{\overset{O}{\|}}{C}-NH_2$, oder $-(CH_2)_2-\overset{\overset{O}{\|}}{C}-NH_2$ ist;

$R_6$ ein Wasserstoffatom, ein Natrium-, Kalium-, Calcium- oder Lithiumion oder der Rest

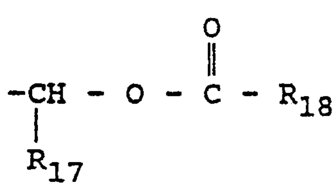 ist;

$R_{17}$ ein Wasserstoffatom, ein gerad- oder verzweigtkettiger Niederalkylrest mit 1 bis 4 Kohlenstoffatomen, eine Cyclohexyl- oder Phenylgruppe ist; und
$R_{18}$ ein Wasserstoffatom oder ein gerad- oder verzweigtkettiger Niederalkylrest mit 1 bis 4 Kohlenstoffatomen ist.

3. Verbindung nach Anspruch 2, wobei:
$R_1$ ein gerad- oder verzweigtkettiger Niederalkylrest mit 1 bis 4 Kohlenstoffatomen oder einer der Reste

$-(CH_2)_r-NH_2$, oder 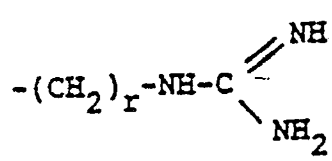 ist;

$R_2$ einer der Reste

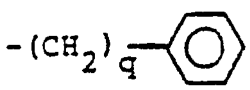 , 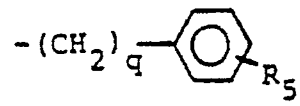 ,

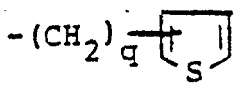 , 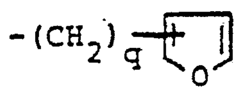 oder 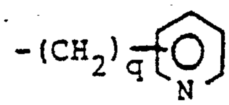 ist;

$R_3$ ein Wasserstoffatom, ein Natrium-, Kalium-, Calcium- oder Lithiumion oder der Rest

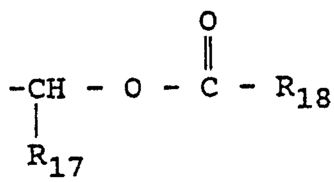 ist;

r eine ganze Zahl von 3 bis 5 ist;
q den Wert 0, 1 oder 2 aufweist;
$R_5$ eine Methyl-, Methoxy- oder Methylthiogruppe, ein Chlor-, Brom- oder ein Fluoratom oder eine Hydroxylgruppe ist;
$R_{17}$ ein Wasserstoffatom, ein gerad- oder verzweigtkettiger Niederalkylrest mit 1 bis 4 Kohlenstoffatomen, eine Cyclohexyl- oder Phenylgruppe ist; und
$R_{18}$ ein Wasserstoffatom oder ein gerad- oder verzweigtkettiger Niederalkylrest mit 1 bis 4 Kohlenstoffatomen ist.

4. Verbindung nach Anspruch 3, wobei
X der Rest

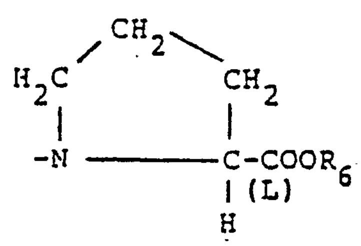

ist; und
$R_6$ ein Wasserstoffatom, ein Natrium-, Kalium-, Calcium-, oder Lithiumion ist.

5. Verbindung nach Anspruch 4, wobei:
$R_1$ eine Methyl- oder $-(CH_2)_4-NH_2$-Gruppe ist;
$R_2$ eine Phenylgruppe ist;
v den Wert 4 aufweist; und
$R_3$ ein Wasserstoffatom, ein Natrium-, Kalium-, Calcium- oder Lithiumion ist.

6. Verbindung nach Anspruch 5, wobei:
$R_1$ eine Methylgruppe ist; und
sowohl $R_3$ als auch $R_6$ Lithium ist.

7. Verbindung nach Anspruch 6, nämlich (S)-1-[2-[[[5-Amino-1-(benzoylamino)pentyl]hydroxyphosphinyl]-oxy]-1-oxopropyl]-L-prolin-dilithiumsalz.

**8.** Verbindung nach Anspruch 5, wobei:
$R_1$ eine $-(CH_2)_4-NH_2$-Gruppe ist; und
$R_3$ und $R_6$ Lithium sind.

**9.** Die Verbindung nach Anspruch 8, nämlich 1-[(S)-6-Amino-2-[[[5-amino-1-(benzoylamino)pentyl]-hydroxyphosphinyl]-oxy]-1-oxohexyl]-L-prolin-dilithiumsalz.

**10.** Pharmazeutische Zusammensetzung, die für die Behandlung von Bluthochdruck in einer säugerart brauchbar ist, umfassend einen pharmazeutisch verträglichen Träger und eine zum Blutdrucksenken wirksame Menge einer Verbindung der Formel

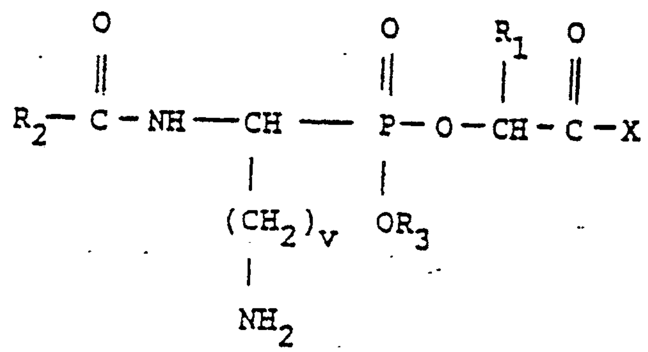

wobei $R_1$, $R_2$, $R_3$, v und x wie in Anspruch 1 definiert sind.

**11.** Pharmazeutische Zusammensetzung, die als analgesisches Mittel brauchbar ist, umfassend einen pharmazeutisch verträglichen Träger und eine analgesisch wirksame Menge einer Enzephalinase-inhibierenden Verbindung der Formel

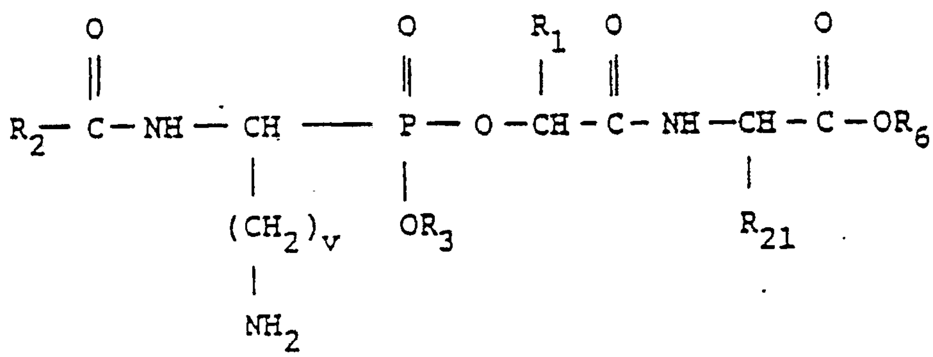

wobei $R_1$, $R_2$, $R_3$, $R_6$, $R_{21}$ und v wie in Anspruch 1 definiert sind.

**12.** Verbindung der Formel

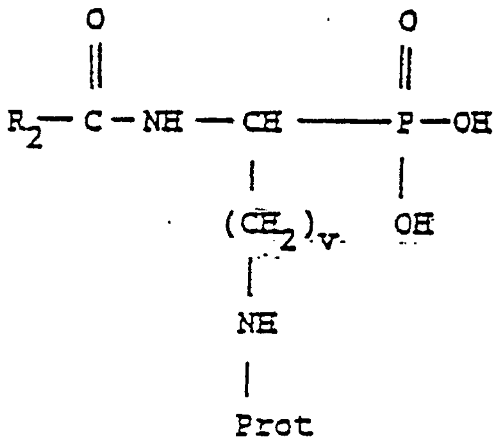

,

wobei:

$R_2$ ein gerad- oder verzweigtkettiger Alkylrest mit 1 bis 10 Kohlenstoffatomen, einer der Reste $-(CH_2)_q$-Cycloalkyl,

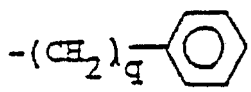,

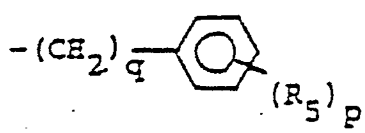, 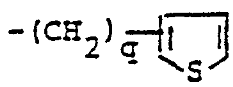,

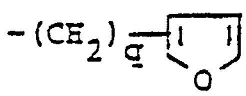 oder 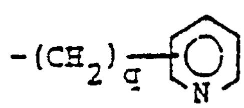 ist;

v eine ganze Zahl von 3 bis 5 ist;

q den Wert 0 hat oder eine ganze Zahl von 1 bis 7 bedeutet;

$R_5$ ein Niederalkylrest mit 1 bis 4 Kohlenstoffatomen, ein Niederalkoxyrest mit 1 bis 4 Kohlenstoffatomen, ein Niederalkylthiorest mit 1 bis 4 Kohlenstoffatomen, ein Chlor-, Brom- oder Fluoratom, eine Trifluormethyl- oder Hydroxylgruppe ist;

p den Wert 1, 2 oder 3 aufweist, mit der Maßgabe, daß p nur dann größer als 1 ist, wenn $R_5$ eine Methyl- oder Methoxygruppe, ein Chlor-, Brom- oder Fluoratom ist; und Prot eine Aminoschutzgruppe ist.

**13.** Verbindung nach Anspruch 12, wobei
$R_2$ einer der Reste

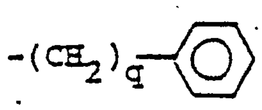 , 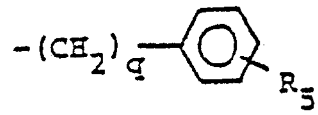 ,

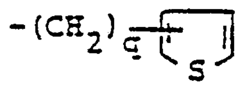 , 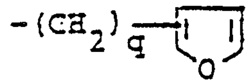

oder 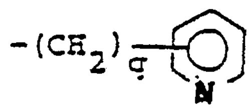 ist;

q den Wert 0, 1 oder 2 aufweist; und
$R_5$ eine Methyl-, Methoxy- oder Methylthiogruppe, ein Chlor-, Brom- oder Fluoratom, oder eine Hydroxylgruppe ist.

**14.** Verbindung nach Anspruch 13, wobei
$R_2$ die Gruppe

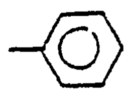 ist;

v den Wert 4 aufweist; und
Prot die Gruppe

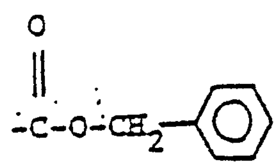 ist.